# EUROPEAN PATENT SPECIFICATION

(11) **EP 0 966 468 B1**
(45) Date of publication and mention of the grant of the patent: **22.09.2004**
(21) Application number: 98912370.8
(22) Date of filing: 23.02.1998
(51) Int. Cl.: C07D 471/06, G01N 31/22, C07D 519/00, C07D 491/22, C09B 5/62, C08K 5/3437

(54) **CROWN ETHER-DERIVATISED PERYLENES**
KRONENETHER DERIVATISIERTE PERYLENE
PERYLENES DERIVES D'ETHER COURONNE

(30) Priority: 05.03.1997 DE 19709004; 05.03.1997 DE 19709008
(43) Date of publication of application: 29.12.1999
(73) Proprietor: Ciba Specialty Chemicals Holding Inc., 4057 Basel (CH)
(72) Inventor: LANGHALS, Heinz, D-85521 Ottobrunn (DE); JONA, Wolfgang, D-84478 Waldkraiburg (DE)
(86) International application number: PCT/EP1998/001023
(87) International publication number: WO 1998/039333

(56) References cited:
- EP-A- 0 000 829
- EP-A- 0 369 733
- WO-A-90/01480
- GB-A- 1 261 984
- GB-A- 1 532 750
- US-A- 4 379 934
- CHEMICAL ABSTRACTS, vol. 72, no. 2, 1970 Columbus, Ohio, US; abstract no. 4335x, NAGASAWA ET AL.: "Perylo[3,4-cd:9,10-c'd']dipyridine pigments" page 54; XP002071092 & JP 06 921 858 A (NIPON) 18 September 1969

## Description

The present invention relates to perylenes of formula I 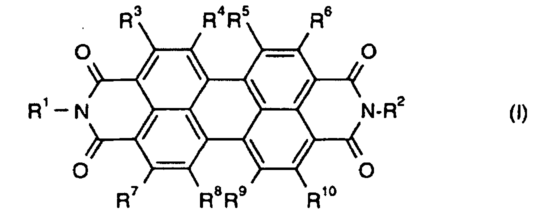 wherein
R¹ is 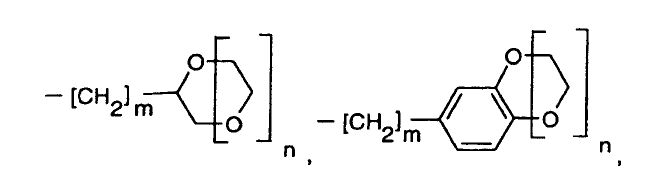 unsubstituted quinolyl, anthrolinyl or phenanthrolinyl or -OH-substituted quinolyl, -OH-substituted anthrolinyl or -OH-substituted phenanthrolinyl, wherein m is an integer from 0 to 4 and n is an integer from 1 to 9,
and the radicals R³ to R¹⁰ are hydrogen or a radical selected from the group consisting of an unsubstituted phenyl or phenyl which is substituted by halogen, cyano, -CN, unsubstituted C₁-C₁₈alkyl or C₁-C₁₈alkyl which is substituted by fluorine, cyano, -OCOR¹⁴, -OR¹², -OCOOR¹⁴, -CON(R¹²)(R¹³) or -OCONHR¹⁴; unsubstituted diphenyl or diphenyl which is substituted by halogen, cyano, -CN, unsubstituted C₁-C₁₈alkyl or C₁-C₁₈alkyl which is substituted by fluorine, cyano, -OCOR¹⁴, -OR¹², -OCOOR¹⁴, -CON(R¹²)(R¹³) or -OCONHR¹⁴; unsubstituted naphthyl or naphthyl which is substituted by halogen, cyano, -CN, unsubstituted C₁-C₁₈alkyl or C₁-C₁₈alkyl which is substituted by fluorine, cyano, -OCOR¹⁴, -OR¹², -OCOOR¹⁴, -CON(R¹²)(R¹³) or -OCONHR¹⁴; an unsubstituted heterocyclic aromatic radical having five to seven ring atoms or substituted heterocyclic aromatic radical having five to seven ring atoms which is substituted by halogen, cyano, -CN, unsubstituted C₁-C₁₈alkyl or C₁-C₁₈alkyl which is substituted by fluorine, cyano, -OCOR¹⁴, -OR¹², -OCOOR¹⁴, -CON(R¹²)(R¹³); halogen; unsubstituted C₁-C₁₈alkyl or C₁-C₁₈alkyl which is substituted by fluorine, cyano, -OCOR¹⁴, -OR¹², -OCOOR¹⁴, -CON(R¹²)(R¹³); -OR¹¹, -CN, -NR¹²R¹³, -COR¹⁴, -NR¹⁵COR¹⁴, -NR¹¹COOR¹⁴, -NR¹¹CONR¹²R¹³, -NHSO₂R¹⁴, -SO₂R¹⁴, -SOR¹⁴, -SO₂OR¹⁴, -CONR¹²R¹³, -SO₂NR¹²R¹³, -N=NR¹⁶, -OCOR¹⁴ and -OCONHR¹⁴, it being possible for two adjacent radicals together to form a carbocyclic or heterocyclic ring, wherein R¹⁴ is C₁-C₁₈alkyl, or C₆-C₁₀aryl, or benzyl that is unsubstituted or substituted by halogen, C₁-C₄alkyl or by C₁-C₄alkoxy, or is a 5- to 7-membered heterocyclic radical,
R¹² and R¹³ are each independently of the other hydrogen, C₁- C₁₈alkyl, C₃- to C₂₄-cycloalkyl, C₆-C₁₀aryl or 5- to 7-membered heteroaryl, which are either unsubstituted or substituted by cyano or by hydroxy, or R¹² and/or R¹³ together with at least one of the other radicals R³ to R¹⁰ form a 5- or 6-membered heterocyclic ring,
R¹¹ is hydrogen, C₁-C₁₈alkyl, C₃- to C₂₄-cycloalkyl, C₆-C₁₀aryl or 5- to 7-membered heteroaryl,
R¹⁵ is hydrogen, C₁-C₁₈alkyl, C₃- to C₂₄-cycloalkyl, C₁-C₄alkylaryl, which are either unsubstituted or substituted by cyano, hydroxy or by C₁-C₄alkoxycarbonyl, C₆-C₁₀aryl, or a 5- to 7-membered heterocycle, which are unsubstituted or substituted by halogen, C₁-C₄alkyl or by C₁-C₄alkoxy,
R¹⁶ is the radical of a coupling component or is C₆-C₁₀aryl that is unsubstituted or substituted by halogen, C₁-C₄alkyl or by C₁-C₄alkoxy, and
R² is one of the radicals mentioned under R¹ or R³ to R¹⁰, with the proviso that R² is not R¹ if R¹ is unsubstituted or substituted quinolyl.

The invention relates also to perylenes II 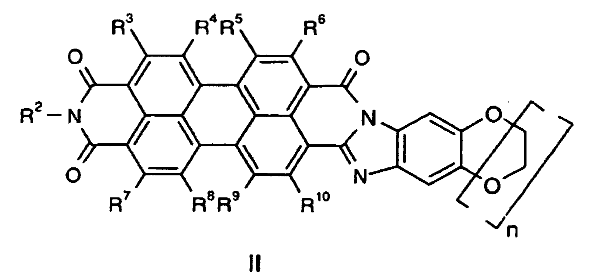 to a process for the preparation of the compounds according to the invention, and to their use.

Crown ethers, that is to say cyclic compounds containing the unit -CH₂-CH₂-O- or similar structural units, are known for their metal-ion-complexing properties. In particular, in contrast to other complexing agents, the complexing of alkali metal and alkaline earth metal cations using crown ethers is usually very successful. However, there has hitherto been no simple and sensitive means of detecting alkali metal and alkaline earth metal cations using fluorescent properties.

Many organic chromophores have hydrophobic properties on account of their π systems. For uses in the aqueous phase, structural elements such as sulfonic acid groups or charges that bring about an effective increase in hydrophilicity are therefore used. Hydrophobic colourants can, however, also be dispersed in water, and the preparation of corresponding colloidal solutions generally requires special techniques, especially when those colloids are to be stored for a relatively long period of time. Flocculation and sedimentation are problems in this connection, which can be counteracted, for example, by the use of protective colloids. However, the introduction of a further substance into the overall system is a disadvantage *(inter alia,* cost, diluting effect).

The problem underlying the present invention is, therefore, to make available novel compounds based on crown ethers that serve as detection reagents for alkali metal and alkaline earth metal cations. Furthermore, the novel compounds are to form thermodynamically stable colloids that can be used as self-dispersing colourants.

Accordingly, the perylenes I defined at the beginning have been found.

There have also been found the perylenes II, processes for the preparation of the compounds according to the invention and their use, *inter alia,* as fluorescent complexing agents for metal ions and self-dispersing organic colourants.

According to the invention, R¹ is 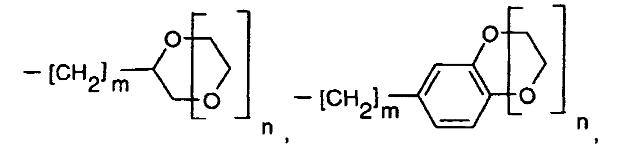 unsubstituted quinolyl, anthrolinyl or phenanthrolinyl or -OH-substituted quinolyl, -OH-substituted anthrolinyl or -OH-substituted phenanthrolinyl, wherein m is an integer from 0 to 4, especially 0, 1, 2, 3 or 4, preferably 0 or 1, and n is an integer from 1 to 9, such as 1, 2, 3, 4, 5, 6, 7, 8, 9, preferably 3, 4 or 5.

Especially preferred radicals R¹ are 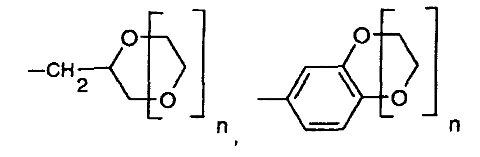 wherein n is 3, 4 or 5, and

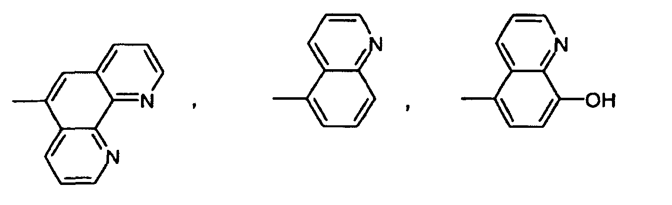

According to the invention, the radicals R³, R⁴, R⁵, R⁶, R⁷, R⁸, R⁹ and R¹⁰ are hydrogen or a radical selected from the group consisting of an unsubstituted phenyl or phenyl which is substituted by halogen, cyano, -CN, unsubstituted C₁-C₁₈alkyl or C₁-C₁₈alkyl which is substituted by fluorine, cyano, -OCOR¹⁴, -OR¹², -OCOOR¹⁴, -CON(R¹²)(R¹³) or -OCONHR¹⁴; unsubstituted diphenyl or diphenyl which is substituted by halogen, cyano, -CN, unsubstituted C₁-C₁₈alkyl or C₁-C₁₈alkyl which is substituted by fluorine, cyano, -OCOR¹⁴, -OR¹², -OCOOR¹⁴, -CON(R¹²)(R¹³) or -OCONHR¹⁴; unsubstituted naphthyl or naphthyl which is substituted by halogen, cyano, -CN, unsubstituted C₁-C₁₈alkyl or C₁-C₁₈alkyl which is substituted by fluorine, cyano, -OCOR¹⁴, -OR¹², -OCOOR¹⁴, -CON(R¹²)(R¹³) or -OCONHR¹⁴; an unsubstituted heterocyclic aromatic radical having five to seven ring atoms or substituted heterocyclic aromatic radical having five to seven ring atoms which is substituted by halogen, cyano, -CN, unsubstituted C₁-C₁₈alkyl or C₁-C₁₈alkyl which is substituted by fluorine, cyano, -OCOR¹⁴, -OR¹², -OCOOR¹⁴, -CON(R¹²)(R¹³); halogen; unsubstituted C₁-C₁₈alkyl or C₁-C₁₈alkyl which is substituted by fluorine, cyano, -OCOR¹⁴, -OR¹², -OCOOR¹⁴, -CON(R¹²)(R¹³); -OR¹¹, -CN, -NR¹²R¹³, -COR¹⁴, -NR¹⁵COR¹⁴, -NR¹¹COOR¹⁴, -NR¹¹CONR¹²R¹³, -NHSO₂R¹⁴, -SO₂R¹⁴, -SOR¹⁴, -SO₂OR¹⁴, -CONR¹²R¹³, -SO₂NR¹²R¹³, -N=NR¹⁶, -OCOR¹⁴ and -OCONHR¹⁴, it being possible for two adjacent radicals together to form a carbocyclic or heterocyclic ring, wherein R¹⁴ is C₁-C₁₈alkyl, or C₆-C₁₀aryl, or benzyl that is unsubstituted or substituted by halogen, C₁-C₄alkyl or by C₁-C₄alkoxy, or is a 5- to 7-membered heterocyclic radical,
R¹¹ is hydrogen, C₁-C₁₈alkyl, C₃- to C₂₄-cycloalkyl, C₆-C₁₀aryl or 5- to 7-membered heteroaryl,
R¹⁵ is hydrogen, C₁-C₁₈alkyl, C₃- to C₂₄-cycloalkyl, C₁-C₄alkylaryl, which are either unsubstituted or substituted by cyano, hydroxy or by C₁-C₄alkoxycarbonyl, C₆-C₁₀aryl or a 5- to 7-membered heterocycle which are either unsubstituted or substituted by halogen, C₁-C₄alkyl or by C₁-C₄alkoxy, and
R¹⁶ is the radical of a coupling component or is C₆-C₁₀aryl that is unsubstituted or substituted by halogen, C₁-C₄alkyl or by C₁-C₄alkoxy.

Unsubstituted or substituted heterocyclic aromatic radical is a mono- to tri-cyclic radical having from five to seven ring atoms. That radical may consist only of at least one heterocyclic ring or the heterocyclic ring(s) may contain at least one fused-on benzene ring. There may be mentioned by way of example pyridyl, pyrimidyl, pyrazinyl, triazinyl, furanyl, pyrrolyl, thiophenyl, quinolyl, isoquinolyl, coumarinyl, benzofuranyl, benzimidazolyl, benzoxazolyl, dibenzofuranyl, benzothiophenyl, dibenzothiophenyl, indolyl, carbazolyl, pyrazolyl, imidazolyl, oxazolyl, isoxazolyl, thiazolyl, indazolyl, benzothiazolyl, pyridazinyl, cinnolinyl, quinazolyl, quinoxalyl, phthalazinyl, phthalazindionyl, phthalimidyl, chromonyl, naphtholactamyl, benzopyridonyl, ortho-sulfobenzimidyl, maleimidyl, naphtharidinyl, benzimidazolonyl, benzoxazolonyl, benzothiazolonyl, benzothiazolinyl, quinazolonyl, pyrimidyl, quinoxalonyl, phthalazonyl, dioxapyrinidinyl, pyridonyl, isoquinolonyl, isothiazolyl, benzisoxazolyl, benzisothiazolyl, indazolonyl, acridinyl, acridonyl, quinazolindionyl, benzoxazindionyl, benzoxazinonyl and phthalimidyl.

The phenyl-, diphenyl-, naphthyl- and/or heterocyclic aromatic radicals are mono- or polysubstituted by customary substituents, especially by substitutents that render the compound insoluble in water. There may be mentioned by way of example:
- halogen, such as fluorine, chlorine, bromine and iodine, preferably chlorine;
- cyano, -CN;
- unsubstituted or substituted C₁-C₁₈alkyl, such as methyl, ethyl, n-propyl, isopropyl, n-butyl, isobutyl, sec-butyl, tert-butyl, tert-amyl, n-hexyl, 1,1,3,3-tetramethylbutyl, n-heptyl, n-octyl, n-nonyl, n-decyl, n-undecyl, n-dodecyl, n-octadecyl, 3-pentyl, 4-heptyl, 5-nonyl, 6-undecyl, 7-tridecyl, 3-hexyl, 3-heptyl, 3-nonyl, 3-undecyl, preferably C₁-C₁₂alkyl, such as methyl, ethyl, n-propyl, isopropyl, n-butyl, sec-butyl, tert-butyl, tert-amyl, n-hexyl, n-heptyl, n-octyl, n-nonyl, n-decyl, n-undecyl, n-dodecyl, 3-pentyl, 4-heptyl, 5-nonyl, 6-undecyl, 7-tridecyl, 3-hexyl, 3-heptyl, 3-nonyl, 3-undecyl, especially C₁-C₈alkyl, such as methyl, ethyl, n-propyl, isopropyl, n-butyl, isobutyl, sec-butyl, tert-butyl, tert-amyl, n-hexyl, 1,1,3,3-tetramethylbutyl, n-heptyl, n-octyl, 3-pentyl, 4-heptyl, 3-hexyl, 3-heptyl, more especially C₁-C₄alkyl, such as methyl, ethyl, n-propyl, isopropyl, n-butyl, isobutyl, sec-butyl, tert-butyl;
   it being possible for the mentioned alkyl groups to be substituted by the following radicals, which generally do not increase hydrophilicity, such as
   fluorine, cyano, -OCOR¹⁴, -OR¹², -OCOOR¹⁴, -CON(R¹²)(R¹³) or -OCONHR¹⁴,
   wherein R¹⁴ is C₁-C₁₈alkyl, such as methyl, ethyl, n-propyl, isopropyl, n-butyl, isobutyl, sec-butyl, tert-amyl, n-hexyl, 1,1,3,3-tetramethylbutyl, n-heptyl, n-octyl, n-nonyl, n-decyl, n-undecyl, n-dodecyl, n-octadecyl, 3-pentyl, 4-heptyl, 5-nonyl, 6-undecyl, 7-tridecyl, 3-hexyl, 3-heptyl, 3-nonyl, 3-undecyl, preferably C₁-C₁₂alkyl, such as methyl, ethyl, n-propyl, isopropyl, n-butyl, sec-butyl, tert-butyl, tert-amyl, n-hexyl, n-heptyl, n-octyl, n-nonyl, n-decyl, n-undecyl, n-dodecyl, 3-pentyl, 4-heptyl, 5-nonyl, 6-undecyl, 7-tridecyl, 3-hexyl, 3-heptyl, 3-nonyl, 3-undecyl; C₆-C₁₀aryl that is unsubstituted or substituted by halogen, such as chlorine or fluorine, C₁-C₄alkyl or by -O-C₁-C₄alkyl, such as phenyl, o-, m- or p-chlorophenyl, o-, m- or p-methylphenyl, 2,5-di-tert-butylphenyl, 1- or 2-naphthyl, preferably phenyl, 1-, 2-naphthyl; or benzyl that is unsubstituted or substituted by halogen, such as chlorine and fluorine, preferably fluorine, C₁-C₄alkyl or by -O-C₁-C₄alkyl; or a 5- to 7-membered heterocyclic radical, such as pyridyl, pyrimidyl, pyrazinyl, triazinyl, furanyl, pyrrolyl, thiophenyl, quinolyl, isoquinolyl, coumarinyl,
   and
   R¹² and R¹³ are hydrogen, C₁-C₁₈alkyl that is unsubstituted or substituted by cyano or by hydroxy, preferably C₁-C₁₂alkyl, especially C₁-C₈alkyl, more especially C₁-C₄alkyl, C₃- to C₂₄-cycloalkyl, preferably C₅-, C₆-, C₁₂-, C₁₅-, C₁₆-, C₂₀- and C₂₄-cycloalkyl, aryl or heteroaryl, preferably derived from the above-mentioned carbocyclic and heterocyclic aromatic radicals, especially phenyl that is unsubstituted or substituted by halogen, C₁-C₄alkyl or by C₁-C₄alkoxy, or R¹² and/or R¹³ together with at least one of the other radicals R³ to R¹⁰ form a 5- or 6-membered hetero ring, for example a pyridine, pyrrole, furan or pyran ring; preferred radicals -OR¹² are hydroxy, -O-methyl, -O-ethyl, -O-isopropyl-, -O-isobutyl, -O-phenyl, -O-2,5-di-tert-butylphenyl; preferred radicals -CON(R¹²)(R¹³) are -CONH₂, -CONMe₂, -CONEt₂, -CON(iPr)₂, -CON(iBu)₂, -CONPh₂, -CON(2,5-di-tert-butylphenyl)₂.
   In a further preferred embodiment there are used as substituents on the alkyl groups mono- or di-alkylated amino, aryl radicals, such as naphthyl or, especially, phenyl that is unsubstituted or substituted by halogen, alkyl or by -O-alkyl, or heterocyclic aromatic radicals, such as 2-thienyl, 2-benzoxazolyl, 2-benzothiazolyl, 2-benzimidazolyl, 6-benzimidazolonyl, 2-, 3- or 4-pyridinyl, 2-, 4- or 6-quinolyl or 1-, 3-, 4-, 6- or 8-isoquinolyl radicals.
   If the mentioned substituents themselves contain alkyl, then that alkyl may be branched or unbranched and may contain preferably from 1 to 18, especially from 1 to 12, more especially from 1 to 8 and very especially from 1 to 4, carbon atoms. Examples of unsubstituted alkyl are methyl, ethyl, n-propyl, isopropyl, n-butyl, sec-butyl, tert-butyl, tert-amyl, n-hexyl, 1,1,3,3-tetramethylbutyl, n-heptyl, n-octyl, n-nonyl, n-decyl, n-undecyl, n-dodecyl, n-octadecyl, 3-pentyl, 4-heptyl, 5-nonyl, 6-undecyl, 7-tridecyl, 3-hexyl, 3-heptyl, 3-nonyl, 3-undecyl. Examples of substituted alkyl are hydroxymethyl, 2-hydroxyethyl, trifluoromethyl, trifluoroethyl, cyanomethyl, methoxycarbonylmethyl, acetoxymethyl and benzyl.
- -OR¹¹, wherein R¹¹ is hydrogen, C₁-C₁₈alkyl as already defined for R¹⁴, including the preferred variants mentioned therefor, C₃- to C₂₄-cycloalkyl, preferably C₅-, C₆-, C₁₂-, C₁₅-, C₁₆-, C₂₀- and C₂₄-cycloalkyl, C₆-C₁₀aryl, such as naphthyl and phenyl, preferably unsubstituted phenyl and phenyl that is substituted by halogen, C₁-C₄alkyl or by C₁-C₄alkoxy, or 5- to 7-membered heteroaryl. There may be mentioned as examples of preferred radicals for R¹¹: methyl, ethyl, n-propyl, isopropyl, n-butyl, sec-butyl, tert-butyl, tert-amyl, n-hexyl, 1,1,3,3-tetramethylbutyl, n-heptyl, n-octyl, n-nonyl, n-decyl, n-undecyl, n-dodecyl, n-octadecyl, 3-pentyl, 4-heptyl, 5-nonyl, 6-undecyl, 7-tridecyl, 3-hexyl, 3-heptyl, 3-nonyl, 3-undecyl, hydroxymethyl, 2-hydroxyethyl, trifluoromethyl, trifluoroethyl, cyanomethyl, methoxycarbonylmethyl, acetoxymethyl, benzyl, phenyl, o-, m- or p-chlorophenyl, o-, m-or p-methylphenyl, 1- or 2-naphthyl, cyclopentyl, cyclohexyl, cyclododecyl, cyclopentadecyl, cyclohexadecyl, cycloeicosanyl, cyclotetracosanyl, thienyl and pyranylmethyl; preferred radicals -OR¹¹ are hydroxy, methoxy, -O-ethyl, -O-isopropyl, -O-isobutyl, -O-phenyl and -O-2,5-di-tert-butylphenyl;
- -NR¹²R¹³, there may be mentioned as examples of preferred radicals: amino, methylamino, dimethylamino, ethylamino, diethylamino, isopropylamino, 2-hydroxyethylamino, 2-hydroxypropylamino, N,N-bis(2-hydroxyethyl)amino, cyclopentylamino, cyclohexylamino, cyclododecylamino, cyclopentadecylamino, cyclohexadecylamino, cycloeicosanylamino, cyclotetracosanylamino, phenylamino, N-methylphenylamino, benzylamino, dibenzylamino, piperidyl and morpholyl;
- -COR¹⁴, there may be mentioned as examples of preferred radicals R¹⁴: methyl, ethyl, n-propyl, isopropyl, n-butyl, sec-butyl, tert-butyl, tert-amyl, n-hexyl, 1,1,3,3-tetramethylbutyl, n-heptyl, n-octyl, n-nonyl, n-decyl, n-undecyl, n-dodecyl, n-octadecyl, 3-pentyl (corresponds to 1-ethyl-n-propyl), 4-heptyl (corresponds to 1-n-propyl-1-n-butyl), 5-nonyl (or: 1-n-butyl-n-pentyl), 6-undecyl (or: 1-n-pentyl-n-hexyl), 7-tridecyl (corresponds to 1-hexylheptyl), 3-hexyl (or: 1-ethyl-n-butyl), 3-heptyl (or: 1-ethyl-n-pentyl), 3-nonyl (or: 1-ethyl-n-heptyl), 3-undecyl (or: 1-ethyl-n-nonyl), hydroxymethyl, 2-hydroxyethyl, trifluoromethyl, trifluoroethyl, cyanomethyl, methoxycarbonylmethyl, acetoxymethyl, benzyl, phenyl, o-, m- or p-chlorophenyl, o-, m- or p-methylphenyl, 1- or 2-naphthyl, cyclopentyl, cyclohexyl, cyclododecyl, cyctopentadecyl, cyclohexadecyl, cycloeicosanyl, cyclotetracosanyl, thienyl, pyranylmethyl and furfuryl;
- -NR¹⁵COR¹⁴, wherein R¹⁵ is hydrogen, C₁-C₁₈alkyl that is unsubstituted or substituted by cyano, hydroxy or by C₁-C₄alkoxycarbonyl, C₃- to C₂₄-cycloalkyl, C₁-C₄alkylaryl, C₆-C₁₀aryl that is unsubstituted or substituted by halogen, C₁-C₄alkyl or by C₁-C₄alkoxy, or a 5- to 7-membered heterocycle, the individual radicals, such as alkyl, alkoxy, aryl, etc., having the meanings defined above for those radicals, including the preferred meanings given therefor. There may be mentioned as examples of radicals: acetylamino, propionylamino, butyrylamino, benzoylamino, p-chlorobenzoylamino, p-methylbenzoylamino, N-methylacetamino, N-methylbenzoylamino, N-succinimido, N-phthalimido and N-(4-amino)phthalimido;
- NR¹¹COOR¹⁴, there may be mentioned as examples of radicals: -NHCOOCH₃, -NHCOOC₂H₅ and -NHCOOC₆H₅;
- -NR¹¹CONR¹²R¹³, there may be mentioned as examples of radicals: ureido, N-methylureido, N-phenylureido and N-(2',4'-dimethyl)phenylureido;
- -NHSO₂R¹⁴, there may be mentioned as examples of radicals: methylsulfonylamino, phenylsulfonylamino, p-tolylsulfonylamino and 2-naphthylsulfonylamino;
- -SO₂R¹⁴, there may be mentioned as examples of radicals: methylsulfonyl, ethylsulfonyl, phenylsulfonyl and 2-naphthylsulfonyl;
- -SOR¹⁴, phenylsulfoxidyl and methylsulfoxidyl may be mentioned as examples of radicals;
- -SO₂OR¹⁴, there may be mentioned as examples of radicals R¹⁴: methyl, ethyl, phenyl, o-, m- or p-chlorophenyl, o-, m- or p-methylphenyl, 1- or 2-naphthyl;
- -CONR¹²R¹³, there may be mentioned as examples of radicals: carbamoyl, N-methylcarbamoyl, N-ethylcarbamoyl, N-phenylcarbamoyl, N,N-dimethylcarbamoyl, N-methyl-N-phenylcarbamoyl, N-1-naphthylcarbamoyl and N-piperidylcarbamoyl;
- -SO₂NR¹²R¹³, there may be mentioned as examples of radicals: sulfamoyl, N-methylsulfamoyl, N-ethylsulfamoyl, N-phenylsulfamoyl, N-methyl-N-phenylsulfamoyl and N-morpholylsulfamoyl;
- -N=NR¹⁶, wherein R¹⁶ is the radical of a coupling component or is a phenyl radical that is unsubstituted or substituted by halogen, alkyl or by -O-alkyl, with halogen and alkyl being as defined above. Alkyl in the definitions of R¹⁶ can have a number of carbon atoms mentioned above as being preferred. There may be mentioned as examples of R¹⁶: the acetoacetarylide, pyrazolyl, pyridonyl, o-, p-hydroxyphenyl, o-hydroxynaphthyl, p-aminophenyl and p-N,N-dimethylaminophenyl radicals.
- -OCOR¹⁴, there may be mentioned as examples of preferred radicals R¹⁴: methyl, ethyl, phenyl, o-, m- or p-chlorophenyl;
- -OCONHR¹⁴, there may be mentioned as examples of preferred radicals R¹⁴: methyl, ethyl, phenyl, o-, m- or p-chlorophenyl.

There may be used as halogen fluorine, chlorine, bromine or iodine, preferably fluorine or chlorine.

There may be used as unsubstituted or substituted C₁-C₁₈alkyl methyl, ethyl, n-propyl, isopropyl, n-butyl, isobutyl, sec-butyl, tert-butyl, tert-amyl, n-hexyl, 1,1,3,3-tetramethylbutyl, n-heptyl, n-octyl, n-nonyl, n-decyl, n-undecyl, n-dodecyl, n-octadecyl, 3-pentyl, 4-heptyl, 5-nonyl, 6-undecyl, 7-tridecyl, 3-hexyl, 3-heptyl, 3-nonyl, 3-undecyl, preferably C₁-C₁₂alkyl, such as methyl, ethyl, n-propyl, isopropyl, n-butyl, see-butyl, tert-butyl, tert-amyl, n-hexyl, n-heptyl, n-octyl, n-nonyl, n-decyl, n-undecyl, n-dodecyl, 3-pentyl, 4-heptyl, 5-nonyl, 6-undecyl, 7-tridecyl, 3-hexyl, 3-heptyl, 3-nonyl, 3-undecyl, especially C₁-C₈alkyl, such as methyl, ethyl, n-propyl, isopropyl, n-butyl, isobutyl, sec-butyl, tert-butyl, tert-amyl, n-hexyl, 1,1,3,3-tetramethylbutyl, n-heptyl, n-octyl, 3-pentyl, 4-heptyl, 3-hexyl, 3-heptyl, more especially C₁-C₄alkyl, such as methyl, ethyl, n-propyl, isopropyl, n-butyl, isobutyl, sec-butyl, tert-butyl;
it being possible for the mentioned alkyl groups to be substituted by the following radicals, which generally do not increase hydrophilicity, such as fluorine, hydroxy, cyano, -OCOR¹⁴, -OR¹², -OCOOR¹⁴, -CON(R¹²)(R¹³) or -OCONHR¹⁴.

In a further preferred embodiment there are used as substituents on the alkyl mono- or di-alkylated amino, aryl radicals, such as naphthyl or, especially, phenyl that is unsubstituted or substituted by halogen, alkyl or by -O-alkyl, or heterocyclic aromatic radicals, such as 2-thienyl, 2-benzoxazolyl, 2-benzothiazolyl, 2-benzimidazolyl, 6-benzimidazolonyl, 2-, 3- or 4-pyridinyl, 2-, 4- or 6-quinolyl or 1-, 3-, 4-, 6- or 8-isoquinolyl radicals.

If the mentioned substituents themselves contain alkyl, then that alkyl may be branched or unbranched and may contain preferably from 1 to 18, especially from 1 to 12, more especially from 1 to 8 and very especially from 1 to 4, carbon atoms. Examples of unsubstituted alkyl are methyl, ethyl, n-propyl, isopropyl, n-butyl, sec-butyl, tert-butyl, tert-amyl, n-hexyl, 1,1,3,3-tetramethylbutyl, n-heptyl, n-octyl, n-nonyl, n-decyl, n-undecyl, n-dodecyl, n-octadecyl, 3-pentyl, 4-heptyl, 5-nonyl, 6-undecyl, 7-tridecyl, 3-hexyl, 3-heptyl, 3-nonyl, 3-undecyl. Examples of substituted alkyl are hydroxymethyl, 2-hydroxyethyl, trifluoromethyl, trifluoroethyl, cyanomethyl, methoxycarbonylmethyl, acetoxymethyl and benzyl.

The radical -OR¹¹ has already been defined above.

There may be used for -NR¹²R¹³ especially the following radicals: amino, methylamino, dimethylamino, ethylamino, diethylamino, isopropylamino, 2-hydroxyethylamino, 2-hydroxypropylamino, N,N-bis(2-hydroxyethyl)amino, cyclopentylamino, cyclohexylamino, cyclododecylamino, cyclopentadecylamino, cyclohexadecylamino, cycloeicosanylamino, cyclotetracosanylamino, phenylamino, N-methylphenylamino, benzylamino, dibenzylamino, piperidyl or morpholyl, special preference being given to dimethylamino, diethylamino, di-n-propylamino, di-n-butylamino, di-n-pentylamino, di-n-hexylamino, di-n-heptylamino, di-n-octylamino, di-n-dodecylamino.

R¹² and/or R¹³, i.e. alone or, where appropriate, together with in each case at least one of the other radicals from the group R³, R⁴, R⁵, R⁶, R⁷, R⁸, R⁹ and R¹⁰, may form one or more 5- or 6-membered, saturated or unsaturated rings, such as pyridine, pyrrole, piperidine, quinoline or benzoquinolizine derivatives.
The radicals -COR¹⁴, -NR¹⁵COR¹⁴, -NR¹¹COOR¹⁴, -NR¹¹CONR¹²R¹³, -NHSO₂R¹⁴, -SO₂R¹⁴, -SOR¹⁴, -SO₂OR¹⁴, -CONR¹²R¹³, -SO₂NR¹²R¹³, -N=NR¹⁶, -OCOR¹⁴ and -OCONHR¹⁴ have already been defined above.

According to the invention, the radical R² is one of the radicals mentioned under R¹ or R³ to R¹⁰, preferably a radical defined under R¹, especially R¹, or a C₁-C₁₈alkyl radical defined under R¹⁴, especially a secondary alkyl radical, such as 1-(C₁-C₉alkyl)-C₂-C₁₀alkyl.

Especially preferred perylenes I are those wherein R³, R⁴, R⁵, R⁶, R⁷, R⁸, R⁹ and R¹⁰ are hydrogen, R² is a secondary alkyl radical, such as 1-(C₁-C₉alkyl)-C₂-C₁₀alkyl, especially those wherein the radical R² has a "swallow-tail" structure, such as 1-methyl-ethyl, 1-ethyl-n-propyl, 1-n-propyl-n-butyl, 1-n-butyl-n-pentyl, 1-n-hexyl-1-heptyl, 1-n-heptyl-1-n-octyl, 1-n-octyl-1-n-nonyl, 1-n-nonyl-1-decyl, or an aromatic radical, especially the phenyl radical, more especially C₁-C₆alkyl-substituted phenyl, such as 2,6-di-tert-butylphenyl and 2,5-di-tert-butylphenyl.

Other preferred perylenes I are those wherein R³, R⁴, R⁵, R⁶, R⁷, R⁸, R⁹ and R¹⁰ are hydrogen, and R² and R¹ are identical meaning 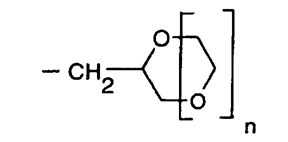 wherein n is preferably 3, 4 or 5.

Other preferred perylenes I are those wherein R³, R⁴, R⁵, R⁶, R⁷, R⁸, R⁹ and R¹⁰ are hydrogen, and R² and R¹ are identical meaning 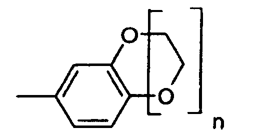 wherein n is preferably 3, 4 or 5.

The following compounds may be mentioned as examples of especially preferred perylenes I: N-(1-hexyl-heptyl)-N'-(5-(8-benzyloxyquinolin)-yl)-perylene-3,4:9,10-bis(dicarboximide), N-(1-nonyl-decyl)-N'-(5-(8-benzyloxyquinolin)-yl)-perylene-3,4:9,10-bis(dicarboximide), N-(1-hexyl-heptyl)-N'-(5-(8-benzyloxyquinolin)-yl)-perylene-3,4:9,10-bis(dicarboximide), N-(1-hexyl-heptyl)-N'-(5-(1,10-phenanthrolin)-yl)-perylene-3,4:9,10-bis(dicarboximide), N-(1-hexyl-heptyl)-N'-(4'-benzo-15-crown-5)-perylene-3,4:9,10-bis(dicarboximide), N-(1-hexylheptyl)-N'-(4'-benzo-18-crown-6)-perylene-3,4:9,10-bis(dicarboximide), N,N'-bis(4'-benzo-15-crown-5)-perylene-3,4:9,10-bis(dicarboximide), N-(1-hexyl-heptyl)-N'-(2-methylene-12-crown-4)-perylene-3,4:9,10-bis(dicarboximide), N-(1-hexyl-heptyl)-N'-(2-methylene-15-crown-5)-perylene-3,4:9,10-bis(dicarboximide), N-(1-hexyl-heptyl)-N'-(2-methylene-18-crown-6)-perylene-3,4:9,10-bis(dicarboximide), bis-N,N'-(2-methylene-12-crown-4)-perylene-3,4:9,10-bis(dicarboximide), bis-N,N'-(2-methylene-15-crown-5)-perylene-3,4:9,10-bis(dicarboximide), bis-N,N'-(2-methylene-18-crown-6)-perylene-3,4:9,10-bis(dicarboximide) and the condensation product of 4',5'-diaminobenzo-15-crown-5 with N-(1-nonyl-decyl)-perylene-3,4:9,10-tetracarboxylic acid 3,4-anhydride 9,10-imide.

The perylenes I according to the invention are preferably obtained by reacting the corresponding perylene anhydrides with the appropriate primary amines in a manner known per se.

To that end it is possible to react on the one hand an unsubstituted or substituted perylene-3,4:9,10-tetracarboxylic acid bisanhydride with an appropriate primary amine, such as a crown ether substituted by an amino group 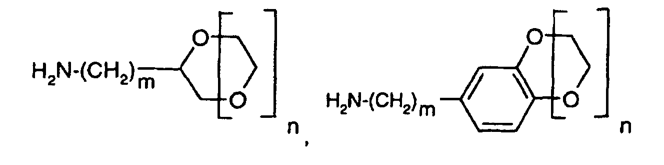 or an appropriately amino-substituted quinolyl, anthrolinyl or phenanthrolinyl radical such as 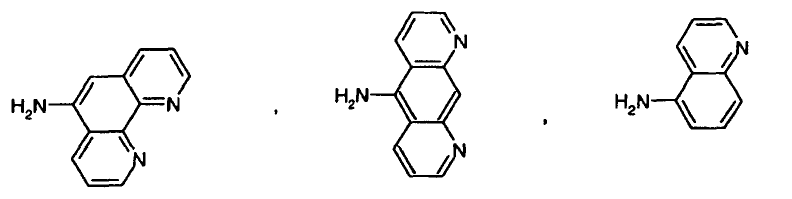 it being possible for the quinolyl, anthrolinyl or phenanthrolinyl radicals in turn to be substituted.

However, it is also possible to use as starting material a substituted or unsubstituted perylene-3,4:9,10-tetracarboxylic acid monoanhydride monoimide, preferably one in which the imide nitrogen atom is substituted by R², R² being especially a secondary alkyl radical having a swallow-tail structure, and to react that perylene anhydride imide with the primary amines mentioned above.

Accordingly, the present invention relates also to a process for the preparation of perylenes I by reacting a perylene bisanhydride or a perylene anhydride imide with a primary amine at elevated temperature, in which process there is used as the primary amine a crown ether, substituted by an amino group, of the general formula III or IV 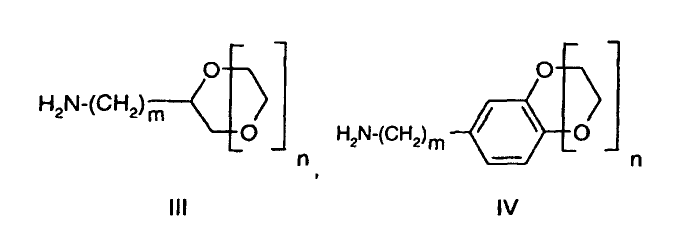 or a phenanthrolinyl radical of e.g. formula V, an anthrolinyl radical of e.g. formula VI or a quinolyl radical of e.g. formula VII 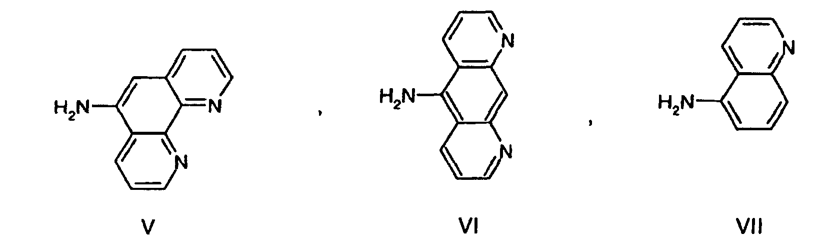 each of which is substituted by an amino group, it being possible for the radicals of formulae V to VII to contain further substituents, if desired.

Perylene-3,4:9,10-tetracarboxylic acid bisanhydrides and perylene-3,4:9,1 0-tetracarboxylic acid monoanhydride monoimides are known or can be prepared according to known methods. For example, the perylene anhydride imides can be obtained by
(a) reacting a perylene bisanhydride with a primary amine, preferably R²-NH₂, or
(b) in a first step reacting a perylene bisanhydride with a primary amine, such as R²-NH₂, to form a perylene bisimide, which is then, in a second step, subjected to hydrolysis, preferably alkaline hydrolysis, to form the corresponding perylene anhydride imide.
Some perylene anhydride imides and their preparation are described, for example, in Chem. Ber. 124 (1991) 529.

The crown ethers substituted by an amino group or the quinolyl, anthrolinyl or phenanthrolinyl radicals, each substituted by an amino group, are likewise known or are obtainable according to known methods from, for example, the corresponding nitro compounds in accordance with customary syntheses (see experimental section), so that further details are unnecessary.

The reaction is preferably carried out at reaction temperatures in a range of from 80 to 200°C, especially from 120 to 180°C. Previous observations have shown that the success of the reaction is not dependent on the choice of pressure range. For the sake of simplicity, the reaction is usually carried out under atmospheric pressure, although lower pressures of up to 10 kPa or pressures up to 10 MPa may be chosen. Depending on the chosen reaction temperature, the reaction times are preferably chosen in the range of from 30 minutes to 6 hours.

In a further preferred embodiment, the reaction is carried out in the presence of a basic organic solvent, preferably a nitrogen-containing heterocycle, such as imidazole, quinoline, pyridine, picoline or N-methylpyrrolidone, especially imidazole or quinoline. The reaction may also be carried out in a glycol, such as ethylene glycol or diethylene glycol. The amount of solvent is generally chosen in the range of from 0.1 to 1 mol per 1 kg of solvent, preferably from 0.1 to 0.8 mol per kg of solvent.

In an especially preferred embodiment, the reaction is carried out in melted imidazole at a temperature in the range of from 100 to 170°C. In a further preferred embodiment, the reaction is carried out under a protecting gas atmosphere. Preferred protecting gases are, for example, nitrogen and the noble gases, such as helium or argon.

The primary amines are generally used in a molar ratio of from 1:1 to 5:1, preferably in a slight excess, especially from 1.05:1 to 1.4:1 (amine:anhydride groups in the perylene used), i.e. starting from a perylene bisanhydride, there is generally used twice the amount of primary amine in comparison with a perylene anhydride imide.

Furthermore, it may be advantageous to carry out the reaction in the presence of heavy metal salts, such as zinc, lead, calcium, copper, manganese, iron, cobalt, nickel, tin, silver or magnesium salts, such as the chlorides, sulfates, nitrates or acetates, especially water-soluble zinc salts, such as zinc acetate and zinc chloride. The molar ratio. of perylene anhydride imide to heavy metal salt is usually chosen in the range of from 10:1 to 1:10, preferably from 4:1 to 1:4.

The perylenes I according to the invention can be purified and isolated by customary methods, such as chromatography, especially column chromatography, or extractive recrystallisation.

The perylenes II according to the invention are preferably obtained analogously to the perylenes mentioned above, by reacting a corresponding perylene anhydride imide with a 4',5'-diaminobenzo-crown ether of formula VIII 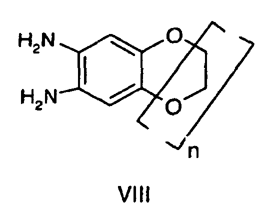 wherein n is preferably 3, 4 or 5.

The corresponding crown ethers are known or are obtainable according to known methods, for example from the corresponding 4'-amino-5'-nitrobenzo-crown ethers (for example, 4'-amino-5'-nitrobenzo-15-crown-5 is commercially available) in a manner known *per se.*

In contrast to the preparation of the perylenes I, it may be advantageous to choose a slightly higher reaction temperature, for example in the range of from 130 to 220°C, preferably from 140 to 200°C.

A further preferred embodiment of the present invention relates to the use of the perylenes I and II according to the invention, preferably those wherein R¹ is

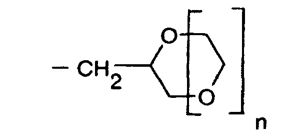

R² is a 1-(C₁-C₉alkyl)-C₂-C₁₀alkyl radical and R³, R⁴, R⁵, R⁶, R⁷, R⁸, R⁹ and R¹⁰ are hydrogen, as detection reagents for heavy metal ions, especially of iron, chromium, nickel, cobalt and copper, especially Fe³⁺ and Cr³⁺, with the exception of manganese. Previous investigations have shown that the complexing of the above-mentioned heavy metal ions, with the exception of manganese, leads to quenching of fluorescence (which can be recognized, for example, in a Stem-Vollmer plot). Using corresponding calibration curves, it is thus possible to detect the mentioned ions quantitatively, manganese generally not interfering with the detection.

A further preferred embodiment of the present invention relates to the use of the perylenes I according to the invention wherein R¹ or R¹ and R² are or contain a benzo-crown ether (in the latter case, m > 0) 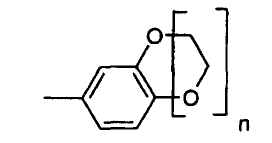 as detection reagents for alkali metal and alkaline earth metal ions. It has been found that the fluorescence of the corresponding benzo-crown ether perylenes according to the invention is quenched by heavy metal ions. The addition of alkali metal or alkaline earth metal ions reverses the quenching, so that the corresponding complexes fluoresce with high quantum yields. By producing calibration curves in a manner known *per se* it is thus possible to detect alkali metal and alkaline earth metal ions quantitatively.

A further embodiment therefore relates also to complexes of the perylenes I and II with heavy metals, such as iron, chromium, nickel, cobalt, copper, titanium, zirconium, hafnium and manganese, and alkali metals, such as sodium and potassium, and alkaline earth metals, such as calcium, magnesium, strontium and barium.

The corresponding complexes can be obtained in a manner known *per se,* for example by adding the perylenes I and II according to the invention to the corresponding heavy metal, alkali metal or alkaline earth metal salts, especially in the form of their nitrates, chlorides, sulfates, hydrogen carbonates or hydrogen phosphates, preferably in the liquid phase, especially in an alcoholic medium, more especially in methanol, ethanol or isopropanol.

Accordingly, a further embodiment relates to a process for the preparation of metal ion complexes of the perylenes I and II according to the invention, by bringing the corresponding metal salts, preferably heavy metal, alkali metal or alkaline earth metal salts, if desired in the liquid phase, into contact with the perylenes I or II.

A further embodiment of the present invention relates to the use of the perylenes I and II according to the invention and of their metal ion complexes as colourants, especially as pigments and dyes, according to methods that are generally in each case known *per se,* preferably
(a) for the mass colouration of polymers, it being possible to use as polymers polyvinyl chloride, cellulose acetate, polycarbonates, polyamides, polyurethanes, polyimides, polybenzimidazoles, melamine resins, silicones, polyesters, polyethers, polystyrene, polymethyl methacrylate, polyethylene, polypropylene, polyvinyl acetate, polyacrylonitrile, polybutadiene, polychlorobutadiene or polyisoprene, and the copolymers of the mentioned monomers;
(b) as vat dyes or mordant dyes, for example for dyeing natural materials and, especially, paper, wood, straw, leather, animal skins or natural fibre materials, such as cotton, wool, silk, jute, sisal, hemp, flax or animal hair (e.g. horsehair) and their conversion products, such as viscose fibres, nitrocellulose or copper rayon (rayon), preferred salts for mordant dyeing being aluminium, chromium and iron salts;
(c) in the preparation of paints, lacquers, especially automotive lacquers, coating compositions, paper dyes, printing inks, inks, especially for use in ink-jet printers, preferably in homogeneous solution as fluorescent inks, and for drawing and writing purposes, and in electrophotography, for example for dry-copying systems (Xerox process) and laser printers;
(d) for security-marking purposes, such as for cheques, cheque cards (credit cards), bank notes, coupons, documents, identity papers and the like, where a special, unmistakable colour impression is to be achieved;
(e) as an additive to colourants, such as pigments and dyes, in which a particular shade of colour is to be achieved; especially luminous shades are preferred;
(f) for labelling objects for the purpose of mechanically recognising those objects by means of the fluorescence, preference being given to the mechanical recognition of objects for sorting, for example for the recycling of plastics, with alphanumerical printing or bar codes preferably being used;
(g) for the frequency conversion of light, for example in order to make longer-wave, visible light from short-wave light, or for frequency doubling or frequency tripling of laser light in non-linear optics;
(h) for the production of passive display elements for a wide variety of display, information and labelling purposes, for example passive display elements, road signs and traffic signals, such as traffic lights;
(i) as starting material for supraconducting organic materials (*via* π-π interactions, after doping with, for example, iodine there is usually obtained an intermediate charge delocalisation);
(j) for solids fluorescent labelling;
(k) for decorative and artistic purposes;
(I) for tracer purposes, for example in biochemistry, medicine, technology and natural science, it being possible to link the colourants according to the invention covalently to substrates or *via* secondary valences, such as hydrogen bonds or hydrophobic interactions (adsorption);
(m) as fluorescent dyes in highly sensitive detection methods (see C. Aubert, J. Fünfschilling, I. Zschokke-Gränacher and H. Langhals, Z. Analyt. Chem. 1985, 320, 361), especially as fluorescent dyes in scintillators;
(n) as dyes or fluorescent dyes in optical light-collecting systems, in fluorescent solar collectors (see H. Langhals, Nachr. Chem. Tech. Lab. 1980, 28, 716), in fluorescence-activated displays (see W. Greubel and G. Baur, Elektronik 1977, 26, 6), in cold light sources for light-induced polymerisation in the preparation of plastics, for materials testing, for example in the manufacture of semiconductor circuitry, for the investigation of microstructures of integrated semiconductor components, in photoconductors, in photographic processes, in display, illumination or image-converting systems in which excitation is effected by means of electrons, ions or UV radiation, for example in fluorescent displays, Braun tubes or in fluorescent tubes, as part of an integrated semiconductor circuit, which contain dyes as such or in conjunction with other semiconductors, for example in the form of an epitaxy, in chemiluminescent systems, for example in chemiluminescent light rods, in luminescent immunoassays or other luminescent detection methods, as highlighter inks, especially for lending visual prominence to writing and drawings or other graphic products, for identifying signs and other objects where a particular visual colour impression is to be achieved, in dye lasers, preferably as fluorescent dyes for producing laser beams and as Q-switches;
(o) as rheology improvers;
(p) as colour filters, and
(q) as dyes in optical memories as information storage material, in such a manner that at elevated temperatures, generally in the range of from 220 to 260°C, preferably from 240 to 250°C, a change in modification occurs, generally recognisable by a change in colour, for example from green-black to red.

The present invention relates also to mass coloured high molecular weight organic material comprising the perylenes I, II according to the invention or a metal ion complex thereof. The colouring is generally carried out by methods known *per se* in accordance with the prior art, so that further details are unnecessary.

### Examples

### Example 1: 5-Amino-8-benzyloxy-quinoline

11.2 g (170.0 mmol) of powdered 85 % potassium hydroxide are introduced, under argon, into 50 ml of DMSO analogously to Chem. Ber. 116 (1983) 2394. While cooling with ice, 11.7 g (50.0 mmol) of 5-amino-8-hydroxyquinoline dihydrochloride are added thereto in small portions, the mixture changing colour to black. After stirring for 20 minutes, 7.5 ml (62.5 mmol) of benzyl bromide are added dropwise in the course of 15 minutes. The mixture is poured onto 100 ml of ice/water, whereupon an ochre-coloured precipitate is formed and is filtered off with suction over a D4 frit and washed with ligroin. The crude product is transferred to a filter thimble and extracted for 3 hours with ligroin in a hot extractor. The olive-green solid remaining in the extraction thimble is recrystallised from a small amount of methanol. The methanol phase is then covered with a layer of ligroin. Yield 4.35 g (35 %) of crude product - Yield after extraction and recrystallisation: 1.0 g (8 %). M.p. 188-189°C. - R_{F} (silica gel/toluene) = 0.10. - IR (KBr): ν = 3422 cm⁻¹ (m), 3305 (m), 3210 (m), 3120 (w), 3080 (w), 2960 (w), 2875 (w), 1641 (m), 1612 (m), 1590 (w), 1581 (m), 1510 (s), 1490 (w), 1476 (s), 1467 (s), 1455 (m), 1419 (m), 1375 (s), 1365 (s), 1285 (s), 1252 (m), 1180 (m), 1092 (s), 1040 (m), 980 (m), 920 (m), 828 (m), 783 (m), 736 (m), 700 (m). UV (DMSO): λₘₐₓ(ε) = 390 nm (3200). C₁₆H₁₄N₂O (250.3): calc.: C 76.78, H 5.64, N 11.19; found: C 76.00, H 5.64, N 11.34.

### Example 2: N-(1-Hexyl-heptyl)-N'-(5-(8-benzyloxyquinolin)-yl)-perylene-3,4:9,10-bis(dicarboximide)

1.72 g (3.00 mmol) of N-(1-hexyl-heptyl)-perylene-3,4:9,10-tetracarboxylic acid 3,4-anhydride 9,10-imide (prepared according to the method described in Chem. Ber. 124 (1991) 529-535 (preparation of compound 2c mentioned therein)), 1.00 g (4.00 mmol) of 5-amino-8-benzyloxyquinoline, 0.26 g (1.20 mmol) of zinc acetate dihydrate and 8 g of imidazole are stirred for 4 hours at 160°C. The reaction is terminated by the addition of 100 ml of ethanol, and the suspension is transferred into 300 ml of 2N hydrochloric acid and stirred for 3 hours. The resulting precipitate is filtered off with suction, washed with water/methanol and then dried at 120°C. Crude product 2.45 g (100 %).

The crude product is purified by chromatography on aluminium oxide (50·4 cm column) using chloroform as eluant. The dye remains firmly adsorbed in the upper third of the column and is then desorbed using the eluant mixture chloroform/1-butanol (40 + 1). The dye fraction is concentrated to dryness by evaporation, taken up in a small amount of chloroform and filtered through a D5 frit. The product is isolated again, pre-dried at 120°C and freed of solvent residues under an oil-pump vacuum at 80°C. Yield 1.21 g (50 %), m.p. 299-300°C. - R_{F} (silica gel/CHCl₃ + ethanol (10 + 1)) = 0.88. - R_{F} (silica gel/CHCl₃ + 1-butanol (40 + 1)) = 0.28. - R_{F} (aluminium oxide/CHCl₃) = 0.30. - IR (KBr) : ν = 2954 cm⁻¹ (m), 2927 (m), 2856 (m), 1708 (s sh), 1698 (s), 1660 (s br.), 1616 (w), 1594 (s), 1579 (m), 1505 (m), 1476 (w), 1432 (w), 1405 (m), 1368 (w), 1343 (s), 1316 (m), 1253 (m br.), 1200 (w), 1176 (w), 1125 (w), 1098 (w), 1015 (w), 960 (w), 855 (w), 811 (m), 790 (w), 747 (m). Fluorescence (CHCl₃): λₘₐₓ(ε) = 540 nm, 578. Diminished fluorescence.
C₅₃H₄₇N₃O₅(806.0): calc. C 78.98, H 5.88, N 5.21; found C 78.76, H 6.00, N 5.12.

### Example 3: N-(1-Nonyl-decyl)-N'-(5-(8-benzyloxyquinolin)-yl)-perylene-3,4:9,10-bis(dicarboximide)

1.58 g (2.40 mmol) of N-(1-nonyl-decyl)-perylene-3,4:9,10-tetracarboxylic acid 3,4-anhydride 9,10-imide (prepared according to the method described in Chem. Ber. 124 (1991) 529-535 (preparation of compound 2e mentioned therein)), 0.90 g (3.60 mmol) of 5-amino-8-benzyloxyquinoline, 0.18 g (0.80 mmol) of zinc acetate dihydrate and 8 g of imidazole. Reaction time 2.5 hours. Purification: chromatography on aluminium oxide/chloroform (50·4 cm column) and twice on silica gel/chloroform/1-butanol (40 + 1). Yield 1.00 g (47 %), m.p. 275-277°C. - R_{F} (silica gel/CHCl₃/ethanol (10 + 1)) = 0.84. IR (KBr): ν = 2955 cm⁻¹ (m), 2926 (s), 2854 (m), 1710 (s sh), 1700 (s br.), 1685 (w), 1660 (s), 1594 (s), 1579 (m), 1506 (m), 1475 (w), 1460 (w), 1457 (w), 1430 (w), 1405 (m), 1368 (w), 1355 (m), 1343 (s), 1316 (m), 1254 (m), 1200 (w), 1177 (m), 1140 (w), 1125 (w), 1110 (w), 1015 (w), 965 (w), 855 (w), 811 (s), 790 (w), 747 (s). - Fluorescence (CHCl₃): λₘₐₓ(ε) = 537 nm, 576. Diminished fluorescence. - C₅₉H₅₉N₃O₅ (890.1): calc. C 79.61, H 6.68, N 4.72; found C 80.05, H 6.68, N 4.73.

### Example 4: N-(1-Hexyl-heptyl)-N'-(5-(8-hydroxyquinolin)-yl)-perylene-3,4:9,10-bis(dicarboximide)

0.40 g (0.50 mmol) of N-(1-hexyl-heptyl)-N'-(5-(8-benzyloxyquinolin)-yl)-perylene-3,4:9,10-bis(dicarboximide) (from Example 2) are heated to boiling in 25 ml of glacial acetic acid. 5 ml of hydrogen bromide (48 % in water) are then added thereto, whereupon a colour change to dark-red can be observed. The reaction is monitored by thin-layer chromatography (silica gel/chloroform/glacial acetic acid (10 + 1)). After 15 minutes, starting material can no longer be detected. After 30 minutes, the reaction is terminated by pouring the mixture into 200 ml of bidistilled water, and the mixture is stirred for a further 30 minutes with gentle heating. The resulting precipitate is filtered off, washed with water/methanol and dried for 16 hours at 110°C. The crude product is purified by extractive recrystallisation twice from distilled toluene. Yield 0.07 g (64 %) after the first recrystallisation, yield 0.03 g (27 %) after the second recrystallisation, m.p. > 300°C. - R_{F} (silica gel/CHCl₃/ethanol (10+1)) = 0.37. - R_{F} (silica gel/CHCl₃/glacial acetic acid (10+1)) = 0.63. - IR (KBr): ν = 3439 cm⁻¹ (s br.), 2958 (m), 2857 (m), 1708 (s sh), 1698 (s), 1660 (s br.), 1594 (s), 1583 (m), 1505 (m), 1477 (m), 1435 (m), 1404 (m), 1375 (m), 1354 (m sh), 1343 (s), 1272 (m), 1254 (m), 1206 (m), 1176 (m), 1130 (w), 970 (w), 855 (w), 838 (w), 811 (s), 795 (w), 747 (m). - UV (CHCl₃): Imax (e) = 528 nm (86 400), 491 (53 100), 460 (19 950). - Fluorescence (CHCl₃): λₘₐₓ(ε) = 538 nm, 573. Diminished fluorescence. - C₄₆H₄₁N₃O₅ (715.9): calc. C 77.18, H 5.77, N 5.87; found C 76.83, H 5.70, N 5.86.

### Example 5: 5-Amino-1,10-phenanthroline

1.0 g (4.4 mmol) of 5-nitro-1,10-phenanthroline is heated to 40°C in 15 ml of absolute ethanol with 0.6 ml (12 mmol) of hydrazine hydrate (100 %). The reduction is started by adding a spatula tip of Pd/C, whereupon considerable evolution of nitrogen can immediately be observed. The reaction is maintained for a further 3 hours by the regular addition of catalyst. The mixture is then heated to reflux in order to concentrate excess hydrazine hydrate. After cooling, the catalyst is filtered off, the solvent is removed *in vacuo* and the crude product is dried for one hour under a water-jet vacuum. 5-Amino-1,10-phenanthroline is used for the following reaction without further purification.

### Example 6: N-(1-Hexyl-heptyl)-N'-(5-(1,10-phenanthrolin)-yl)-perylene-3,4:9,10-bis(dicarboximide)

1.72 g (3.00 mmol) of N-(1-hexyl-heptyl)-perylene-3,4:9,10-tetracarboxylic acid 3,4-anhydride 9,10-imide and 0.87 g (4.40 mmol) of unpurified 5-amino-1,10-phenanthroline are reacted for 3 hours at 150°C in 10 g of imidazole with the addition of 0.10 g (0.45 mmol) of zinc acetate dihydrate. The reaction product is introduced with about 50 ml of ethanol into 300 ml of 2N hydrochloric acid and stirring is carried out for 2 hours at room temperature. The resulting precipitate is filtered off, washed with water/methanol and dried for 16 hours at 120°C. The crude product can first be separated over aluminium oxide/chloroform/ethanol (10 + 1) into two rough fractions A and B. Fraction A is discarded. The main fraction B is first applied to a column of silica gel/chloroform/glacial acetic acid (10 + 1). It is thus possible to elute some of the impurities, while N-(1-hexyl-heptyl)-N'-(5-(1,10-phenanthrolin)-yl)-perylene-3,4:9,10- bis(dicarboximide) remains firmly adsorbed on the material of the column. Desorption of N-(1-hexyl-heptyl)-N'-(5-(1,10-phenanthrolin)-yl)-perylene-3,4:9,10-bis(dicarboximide) is carried out with the eluant mixture chloroform/triethylamine (10 + 1). Yield 0.65 g, m.p. > 350°C. - R_{F} (silica gel/CHCl₃/triethylamine (10 + 1)) = 0.58. - IR (KBr): ν = 2955 cm⁻¹ (m), 2928 (m), 2857 (m), 1710 (m, sh), 1698 (s), 1658 (s, br.), 1596 (s), 1579 (m), 1510 (w), 1438 (w), 1431 (w), 1405 (m), 1368 (w, sh), 1355 (m, sh), 1344 (s), 1253 (m), 1205 (w), 1176 (m), 1110 (w), 968 (w), 855 (w), 830 (w), 811 (m), 748 (m), 742 (m), 730 (w), 690 (w). - C₄₉H₄₂N₄O₄: calc. 750.3206; found 750.3208 (MS). - C₄₉H₄₂N₄O₄ (750.9): calc. C 78.38, H 5.64, N 7.46; found C 76.85, H 5.61, N 7.68.

### Example 7: N-(1-Hexyl-heptyl)-N'-(4'-benzo-15-crown-5)-perylene-3,4:9,10-bis(dicarboximide)

0.70 g (1.22 mmol) of N-(1-hexyl-heptyl)-perylene-3,4:9,10-tetracarboxylic acid 3,4-anhydride 9,10-imide and 0.43 g (1.52 mmol) of 4'-aminobenzo-15-crown-5 are heated for 4 hours at 140°C, under a protecting gas, with 0.08 g (0.36 mmol) of zinc acetate dihydrate in 10 g of imidazole. Before solidifying, the mixture is introduced with a total of 100 ml of ethanol into 300 ml of 2N hydrochloric acid and stirred for 2 hours at room temperature. The resulting precipitate is filtered off with suction over a D4 frit, washed with water and dried. The filtrate is extracted with a total of 800 ml of chloroform, the solvent is removed and the two crude products are combined. The colourant so obtained is worked up by column chromatography on aluminium oxide in chloroform/ethanol (20 + 1) (50.4 cm column), the product N-(1-hexyl-heptyl)-N'-(4'-benzo-15-crown-5)-perylene-3,4:9,10-bis(dicarboximide) being obtained as the first red, non-fluorescent band. The dye fraction obtained is filtered through a D4 frit and the solvent is then removed; the fraction is washed with methanol/water and then dried for 24 hours under an oil-pump vacuum (80°C/0.13 mbar). Yield 0.51 g (50 %), m.p. 288-290°C. - R_{F} (silica gel/CHCl₃ + ethanol (10 + 1)) = 0.25. IR (KBr): ν = 2955 cm⁻¹ (m), 2927 (s), 2858 (m), 1698 (s), 1660 (s), 1594 (s), 1579 (m), 1512 (s), 1455 (w), 1433 (w), 1405 (m), 1354 (m), 1345 (s br.), 1308 (w), 1264 (m), 1254 (s br.), 1176 (w), 1130 (s br.), 980 (w), 855 (w), 811 (s), 795 (m), 746 (m).- Fluorescence (CHCl₃): λₘₐₓ(ε) = 535 nm, 575. C₅₁H₅₄N₂O₉ (838.4): calc. C 73.01, H 6.49, N 3.34; found C 73.12, H 6.46, N 3.14.

### Example 8: 4'-Aminobenzo-18-crown-6

0.52 g (1.45 mmol) of 4'-nitrobenzo-18-crown-6 and 0.18 g (3.60 mmol) of hydrazine hydrate are dissolved in 10 ml of absolute ethanol and heated to 40°C. The catalyst Pd/C (10 % Pd) is then added in portions until no further evolution of nitrogen can be observed. The reaction mixture is then heated for one hour under reflux at boiling point and cooled to room temperature, and the solution is filtered off under a protecting gas. Following that procedure, all the components that are volatile at room temperature are removed under an oil-pump vacuum. The crude product is used for the following reactions without further purification.

### Example 9: N-(1-Hexyl-heptyl)-N'-(4'-benzo-18-crown-6)-perylene-3,4:9,10-bis(dicarboximide)

0.83 g (1.45 mmol) of N-(1-hexyl-heptyl)-perylene-3,4:9,10-tetracarboxylic acid 3,4-anhydride 9,10-imide, 0.10 g (0.46 mmol) of zinc acetate dihydrate and 3 g of imidazole are added to the colourless oil of Example 8 (4'-aminobenzo-18-crown-6). The mixture is stirred for 3 hours at 140°C and is then diluted with 25 ml of ethanol and introduced into 200 ml of 2N hydrochloric acid. After stirring for one hour at room temperature, the resulting precipitate is filtered off with suction, washed until neutral with water/methanol and dried at 110°C in a drying cabinet. When working up by chromatography on aluminium oxide (60.4 cm column)/chloroform/ethanol (10 + 1) is carried out, two main fractions are isolated. The first fraction is discarded. The second main fraction is chromatographed on silica gel/chloroform/glacial acetic acid (10 + 1), the product N-(1-hexyl-heptyl)-N'-(4'-benzo-18-crown-6)-perylene-3,4:9,10-bis(dicarboximide) remaining adsorbed in the upper third of the column. The dye fraction is eluted as a deep-red, non-fluorescent band using chloroform/triethylamine (10 + 1), concentrated using a rotary evaporator, washed with water, dried at 110°C and prepared in the manner customary for elemental analysis. Yield 0.25 g (20 %), m.p. 247-249°C. - R_{F} (silica gel/CHCl₃/triethylamine (10+1)) = 0.82. - IR (KBr) : ν = 2955 cm⁻¹ (m), 2927 (m), 2857 (m), 1696 (s), 1658 (s), 1594 (s), 1579 (m), 1514 (m), 1457 (w), 1433 (w), 1405 (w), 1355 (m), 1345 (s), 1255 (s), 1197 (w), 1177 (m), 1123 (s), 960 (w), 811 (m), 796 (w), 746 (m). - Fluorescence (CHCl₃): λₘₐₓ(ε) = 538 nm, 577. - C₅₃H₅₈N₂O₁₀ (883.1): calc. C 72.09, H 6.62, N 3.17; found C 71.46, H 6.77, N 3.29. - C₅₃H₅₈N₂O₁₀ · 0.5 H₂O (892.1): calc. C 71.36, H 6.66, N 3.14; found C 71.46, H 6.77, N 3.29.

### Example 10: N,N'-Bis(4'-benzo-15-crown-5)-perylene-3,4:9,10-bis(dicarboximide)

0.39 g (1.27 mmol) of perylene-3,4:9,10-tetracarboxylic acid bisanhydride, 0.76 g (2.67 mmol) of 4-aminobenzo-15-crown-5, 0.08 g of zinc acetate dihydrate and 7 g of imidazole are stirred for 3 hours at 140°C. The reaction mixture is suspended while still warm in 50 ml of ethanol and is then transferred into 300 ml of 2N hydrochloric acid. After one hour, the resulting precipitate is filtered off with suction and dried. 0.5 g of the crude product is boiled a total of three times with 200 ml of 10 % potassium carbonate solution each time and the insoluble product is isolated using a D4 frit. The filtrate is discarded. The precipitate is washed several times with hot water and then dried at 100°C. The powdered product N,N'-bis(4'-benzo-15-crown-5)-perylene-3,4:9,10-bis(dicarboximide) is transferred to an extraction thimble and extracted in succession with methylene chloride, acetone and ethanol for in each case from one to two days. The insoluble product N,N'-bis(4'-benzo-15-crown-5)-perylene-3,4:9,10-bis(dicarboximide) is in the extraction thimble. Yield 0.11 g (22 %), based on 0.5 g of the crude product treated in that manner. - IR (KBr) : ν = 2920 cm⁻¹ (m), 2870 (m), 1702 (s), 1662 (s), 1594 (s), 1578 (m), 1514 (s), 1453 (m), 1431 (m), 1404 (m), 1362 (s), 1331 (m), 1268 (s br.), 1256 (s br.), 1197 (m), 1179 (m), 1133 (m br.), 1122 (s br.), 1050 (m), 970 (w), 940 (m), 856 (m), 811 (m), 798 (m), 768 (m), 760 (m), 630 (m). - UV (H₂SO₄): λₘₐₓ(ε) = 603 nm (79 000), 558 (46 200), 522 sh (16 600), 400 (6500). - C₅₂H₄₆N₂O₁₄ (922.9): calc. C 67.67, H 5.02, N 3.03; found C 62.97, H 4.59, N 2.91.

### Example 11: N-(1-Hexyl-heptyl)-N'-(2-methylene-12-crown-4)-perylene-3,4:9,10-bis(dicarboximide)

0.57 g (1.00 mmol) of N-(1-hexyl-heptyl)-perylene-3,4:9,10-tetracarboxylic acid 3,4-anhydride 9,10-imide and 0.25 g (1.22 mmol) of 2-aminomethyl-12-crown-4 are maintained at 140°C for one hour in 4 g of imidazole. Before cooling, the reaction mixture is introduced with 50 ml of ethanol into 200 ml of 2N hydrochloric acid. After stirring for 2 hours, the resulting precipitate is filtered off with suction over a D4 frit, washed with methanol/water and dried overnight at 110°C. The crude product is worked up by column chromatography on aluminium oxide (30.4 cm column)/chloroform/ethanol (10 + 1). The product is eluted with the eluant mixture chloroform/ethanol (10 + 1). Yield 0.53 g (70 %). The dye N-(1-hexylheptyl)-N'-(2-methylene-12-crown-4)-perylene-3,4:9,10-bis(dicarboximide) is highly purified by chromatography on silica gel/chloroform/ethanol (20 + 1). The dye fraction is filtered through a D4 frit, methanol is added, and the mixture is concentrated by evaporation and dried for 24 hours at 90°C under an oil-pump vacuum. Yield 0.36 g (47 %), m.p. 251-252°C. - R_{F} (silica gel/CHCl₃/ethanol (10+1)) = 0.56. - IR (KBr): ν = 2953 cm⁻¹ (m), 2927 (m), 2855 (m), 1696 (s), 1658 (s), 1595 (s), 1577 (m), 1506 (w), 1437 (m), 1405 (m), 1355 (m), 1344 (s br.), 1252 (m), 1177 (w), 1127 (m), 1098 (w), 860 (w), 810 (m), 747 (m). - UV (CHCl₃): λₘₐₓ(ε) = 527 nm (86 100), 490 (51 800), 459 (19 400). - Fluorescence (CHCl₃): λₘₐₓ(ε) = 535 nm, 572. - Solid-state fluorescence: λₘₐₓ(ε) = 528 nm, 569, 603. - C₄₆H₅₂N₂O₉ (760.9): calc. C 72.61, H 6.89, N 3.68; found C 72.74, H 6.57, N 3.79.

### Example 12: N-(1-Hexyl-heptyl)-N'-(2-methylene-15-crown-5)-perylene-3,4:9,10-bis(dicarboximide)

Under argon, 0.50 g (0.87 mmol) of N-(1-hexyl-heptyl)-perylene-3,4:9,10-tetracarboxylic acid 3,4-anhydride 9,10-imide is reacted at 140°C for one hour with 0.25 g (1.00 mmol) of 2-aminomethyl-15-crown-5 in 4 g of imidazole. Before cooling, the reaction product is suspended in 50 ml of ethanol; 200 ml of 2N hydrochloric acid are added and the mixture is then stirred for 2 hours at room temperature. The resulting precipitate is filtered off with suction over a D4 frit, washed (methanol/water) and dried for 8 hours at 100°C. For purification, the crude product is chromatographed on aluminium oxide (30·4 cm column) and twice on silica gel, in each case using chloroform/ethanol (10 + 1) as eluant. The dye fraction so obtained is filtered through a D4 frit, the solvent is removed and drying is carried out for 24 hours at 100°C under an oil-pump vacuum. Yield 0.57 g (81 %) of crude product. - Yield 0.51 g (72 %), m.p.190-192°C. - R_{F} (silica gel/CHCl₃/ethanol (10+1)) = 0.27. - IR (KBr): ν = 2950 cm⁻¹ (m), 2927 (m), 2852 (m), 1697 (s), 1658 (s), 1595 (s), 1576 (m), 1506 (w), 1438 (m), 1404 (m), 1382 (w), 1351 (m), 1343 (s br.), 1252 (m), 1175 (w), 1118 (m br.), 942 (w), 855 (w), 811 (m), 747 (m).- UV (CHCl₃): λₘₐₓ(ε) = 527 nm (84 600), 489 (50 700), 459 (18 200). - Fluorescence (CHCl₃): λₘₐₓ(ε) = 534 nm, 574. C₄₈H₅₆N₂O₉ (805.0): calc. C 71.62, H 7.01, N 3.48; found C 71.96, H 7.03, N 3.51.

### Example 13: N-(1-Hexyl-heptyl)-N'-(2-methylene-18-crown-6)-perylene-3,4:9,10-bis(dicarboximide)

0.29 g (0.50 mmol) of N-(1-hexyl-heptyl)-perylene-3,4:9,10-tetracarboxytic acid 3,4-anhydride 9,10-imide and 0.15 g (0.51 mmol) of 2-aminomethyl-18-crown-6 in 5 g of imidazole are reacted for one hour, with stirring, at an oil bath temperature of 140°C. Before cooling, the mixture is suspended in 50 ml of ethanol; 100 ml of 2N hydrochloric acid are added and the mixture is stirred overnight at room temperature. The resulting precipitate is filtered off with suction over a D4 frit, washed (water/methanol) and dried for 16 hours at 110°C. The dye crude product is purified by column chromatography on aluminium oxide (30.4 cm column) using chloroform/ethanol (10 + 1) as eluant. The product N-(1-hexyl-heptyl)-N'-(2-methylene-18-crown-6)-perylene-3,4:9,10-bis(dicarboximide) firmly adsorbed on the silica gel is eluted with chloroform/triethylamine (20 + 1). The main fraction is filtered through a D4 frit, methanol is added, the mixture is concentrated by evaporation and the residue is dried for 24 hours at 90°C under an oil-pump vacuum. Yield 0.24 g (56 %), m.p. 161-163°C. - R_{F} (silica gel/CHCl₃/triethylamine (10+1)) = 0.87. - R_{F} (silica gel/CHCl₃/triethylamine (20+1)) = 0.51. IR (KBr): ν = 2952 cm⁻¹ (m), 2927 (s), 2859 (m), 1697 (s), 1682 (w), 1656 (s), 1634 (w), 1595 (s), 1576 (m), 1439 (m), 1404 (m), 1351 (s), 1344 (s), 1251 (m), 1175 (w), 1106 (s br.), 960 (w), 860 (w), 810 (m), 747 (m). - UV (CHCl₃): λₘₐₓ(ε) = 526 nm (84 000), 490 (50 300), 459 (18 000). - Fluorescence (CHCl₃): λₘₐₓ(ε) = 535 nm, 575. C₅₀H₆₀N₂O₁₀ (849.0): calc. C 70.73, H 7.12, N 3.30; found C 70.98, H 7.17, N 3.42.

### Example 14: Addition of various heavy metal ions to the dye N-(1-hexyl-heptyl)-N'-(2-methylene-15-crown-5)-peryfene-3,4:9,10-bis(dicarboximide)

Weighed-in amount of the dye N-(1-hexyl-heptyl)-N'-(2-methylene-15-crown-5)-perylene-3,4:9,10-bis(dicarboximide) (M = 805.0 g mol⁻¹): 1.315 mg in 100 ml of chloroform. - 3 ml (4.9·10⁻⁵ mmol) of the dye solution are placed in each of a number of vessels; an increasing amount of the heavy metal salt is added and the mixture is made up to a constant volume. The sealed vessels are stored for 3-7 days with the exclusion of light so that the metal can become thoroughly bonded by the crown ether.

The quenching of fluorescence by an added quencher is generally described by the Stern-Vollmer equation, it being possible, if the distribution function of the absorption and fluorescence spectrum does not change, to replace the ratio of the quantum yields Q_{f}⁰/Q_{f} by the ratio of the fluorescence intensities F₀/F (F₀ = fluorescence intensity in the absence of a quencher, F = fluorescence intensity with a quencher), which is readily obtainable by experiment. For linearisation, F₀/F can then be plotted against the quencher concentration [Q].

### (a) Iron(III) nitrate nonahydrate

Weighed-in amount of iron(III) nitrate nonahydrate (M = 404.0 g mol⁻¹): 0.596 mg in 25 ml of ethanol, or 1 ml of solution accordingly contains 5.9·10⁻⁵ mmol of Fe³⁺ salt.

**Table 1:**

| Fluorescence quenching of N-(1-hexyl-heptyl)-N'-(2-methylene-15-crown-5)-perylene-3,4:9,10-bis(dicarboximide) (4.9·10⁻⁸ mol/litre) by iron(III) nitrate nonahydrate | | | | |
|---|---|---|---|---|
| ml Fe³⁺ | [Fe³⁺] | [Dye] : [Fe³⁺] | Fᵣₑₗ | F₀/F |
| solution | [10⁻⁸ mol/litre] | | 538 nm | |
| 0 | 0 | -- | 687 | 1.0 |
| 0.1 | 0.59 | 8 : 1 | 646 | 0.94 |
| 0.2 | 1.18 | 4 :1 | 631 | 0.92 |
| 0.4 | 2.36 | 2 : 1 | 618 | 0.90 |
| 0.6 | 3.54 | 1.4 : 1 | 615 | 0.89 |
| 0.8 | 4.72 | 1 : 1 | 631 | 0.91 |
| 1.0 | 5.9 | 1 : 1.2 | 657 | 0.95 |
| 1.5 | 8.85 | 1 : 1.8 | 573 | 0.83 |
| 2.0 | 11.8 | 1 : 2.4 | 560 | 0.82 |
| 4.0 | 23.6 | 1 : 4.8 | 488 | 0.71 |
| 8.0 | 47.2 | 1 : 9.6 | 375 | 0.55 |

### (b) Chromium(III) nitrate nonahydrate

Weighed-in amount of chromium(III) nitrate nonahydrate (M = 400.1 g mol⁻¹): 0.752 mg in 25 ml of ethanol. 1 ml of solution accordingly contains 7.51·10⁻⁸ mol of Cr³⁺ salt.

**Table 2:**

| Fluorescence quenching of N-(1-hexyl-heptyl)-N'-(2-methylene-15-crown-5)-perylene-3,4:9,10-bis(dicarboximide) (4.9·10⁻⁸ mol/litre) by chromium(III) nitrate nonahydrate | | | | |
|---|---|---|---|---|
| ml Cr³⁺ | [Cr³⁺] | [Dye] : [Cr³⁺] | Fᵣₑₗ | F₀/F |
| solution | [10⁻⁸ mol/litre] | | 538 nm | |
| 0 | 0 | -- | 797 | 1.0 |
| 0.1 | 0.75 | 6.5 : 1 | 795 | 0.99 |
| 0.2 | 1.50 | 3.3 : 1 | 733 | 0.92 |
| 0.3 | 2.25 | 2.2 : 1 | 737 | 0.92 |
| 0.4 | 3.0 | 1.6 : 1 | 708 | 0.89 |
| 0.5 | 3.75 | 1.3 : 1 | 698 | 0.88 |
| 0.6 | 4.5 | 1.1 : 1 | 684 | 0.86 |
| 0.8 | 6.0 | 1 : 1.2 | 680 | 0.85 |
| 1.0 | 7.5 | 1 : 1.5 | 660 | 0.83 |
| 2.0 | 15 | 1 : 3 | 658 | 0.83 |
| 4.0 | 30 | 1 : 6 | 543 | 0.68 |

### (c) Manganese(II) chloride tetrahydrate

Weighed-in amount of manganese(II) chloride tetrahydrate (M = 197.9 g mol⁻¹): 1.566 mg in 25 ml of ethanol. 1 ml of solution accordingly contains 3.17·10⁻⁷ mol of Mn²⁺ salt.

**Table 3:**

| Fluorescence quenching of N-(1-hexyl-heptyl)-N'-(2-methylene-15-crown-5)-perylene-3,4:9,10-bis(dicarboximide) (4.9·10⁻⁸ mol/litre) by manganese(II) chloride tetrahydrate | | | | |
|---|---|---|---|---|
| ml Mn²⁺ | [Mn²⁺] | [Dye] : [Mn²⁺] | Fᵣₑₗ | F₀/F |
| solution | [10⁻⁸ mol/litre] | | 538 nm | |
| 0 | 0 | -- | 691 | 1.00 |
| 0.01 | 0.32 | 15: 1 | 678 | 1.02 |
| 0.02 | 0.64 | 7 : 1 | 678 | 1.02 |
| 0.03 | 0.96 | 5 : 1 | 673 | 1.03 |
| 0.04 | 1.28 | 3.8 : 1 | 648 | 1.07 |
| 0.05 | 1.6 | 3 : 1 | 699 | 0.99 |
| 0.06 | 1.92 | 2.5 : 1 | 683 | 1.01 |
| 0.08 | 2.56 | 1.9 : 1 | 718 | 0.96 |
| 0.1 | 3.2 | 1.5 : 1 | 736 | 0.94 |
| 0.4 | 12.8 | 1 : 2.6 | 705 | 0.98 |
| 1.0 | 32 | 1 : 6.5 | 707 | 0.98 |

### Example 15: Bis-N,N'-(2-methylene-12-crown-4)-perylene-3,4:9,10-bis(dicarboximide)

A mixture of 0.20 g (0.50 mmol) of perylenetetracarboxylic acid bisanhydride and 0.22 g (1.07 mmol) of 2-aminomethyl-12-crown-4 in 4 g of imidazole is reacted at 140°C. After 1.5 hours, 50 ml of ethanol are added carefully; 200 ml of 2N hydrochloric acid are added and the mixture is stirred for 2 hours at room temperature. The resulting precipitate is isolated over a D4 frit. The filtrate is extracted repeatedly with a total of 200 ml of chloroform and, after the solvent has been evaporated off, the solid phases are combined. Yield: 0.32 g (82 %) of crude product.

After concentration, the suspension of bis-N,N'-(2-methylene-12-crown-4)-perylene-3,4:9,10-bis(dicarboximide) obtained above is allowed to stand in chloroform (with ethanol or triethylamine) in the closed vessel for from one to three days. Some of the impurities can be separated off by chromatography on silica gel using chloroform/ethanol (10 + 1). Since some of the product bis-N,N'-(2-methylene-12-crown-4)-perylene-3,4:9,10-bis(dicarboximide) remains adsorbed on the silica gel and, moreover, migrates as a very broad, unstructured band; the latter is focused with the eluant mixture chloroform/triethylamine (10 + 1) and the dye fraction is separated. The solution is concentrated by evaporation, the solid material is taken up in chloroform and filtered through a D4 frit, and ligroin is added. After the solvent mixture has been distilled off, the black product bis-N,N'-(2-methylene-12-crown-4)-perylene-3,4:9,10-bis(dicarboximide) is washed with diethyl ether and pentane. For the elemental analysis, 0.1 g of bis-N,N'-(2-methylene-12-crown-4)-perylene-3,4:9,10-bis(dicarboximide) is purified further by extractive recrystallisation from distilled toluene. Yield 0.16 g (41 %) of a greenish-black microcrystalline powder, m.p. > 360°C.

At a temperature of 240-250°C, the black modification changes into a red modification.

R_{F} (silica gel/CHCl₃/ethanol (10+1)) = 0.02. - IR (KBr): ν = 2922 cm⁻¹ (m br.), 2868 (m), 1694 (s), 1658 (s br.), 1595 (s), 1578 (m), 1506 (w), 1441 (m), 1403 (m), 1360 (m br.), 1347 (m), 1300 (w), 1252 (m), 1185 (w), 1129 (m), 1094 (m), 1030 (w), 915 (w), 860 (w), 810 (m), 795 (w), 747 (m). - C₄₂H₄₂N₂O₁₂ (766.8): calc. C 65.79, H 5.52, N 3.65; found C 65.66, H 5.51, N 3.65.

### Example 16: Bis-N,N'-(2-methylene-15-crown-5)-perylene-3,4:9,10-bis(dicarboximide)

0.35 g (0.90 mmol) of perylenetetracarboxylic acid bisanhydride and 0.45 g (1.80 mmol) of 2-amino-methyl-15-crown-5 are reacted for 2 hours at an oil bath temperature of 140°C in 5 g of imidazole. The reaction mixture is taken up in 200 ml of chloroform and is extracted in order to remove imidazole once with 100 ml of concentrated hydrochloric acid and twice with 100 ml of 2N hydrochloric acid each time. Bis-N,N'-(2-methylene-15-crown-5)-perylene-3,4:9,10-bis(dicarboximide) goes into a colloidal solution, and the combined hydrochloric acid phases are therefore extracted again with a total of 300 ml of chloroform. The red solid, which is moderately soluble in both phases, is worked up together with the organic phase. The combined chloroform extracts are concentrated to a volume of about 20 ml and dried and purified by chromatography on aluminium oxide (50.4 cm column, chloroform/ethanol (20 + 1)). The dye fraction so obtained is then chromatographed on silica gel using chloroform/triethylamine (10 + 1) as eluant. Bis-N,N'-(2-methylene-15-crown-5)-perylene-3,4:9,10-bis(dicarboximide) is concentrated to dryness by evaporation, taken up in a small amount of chloroform and filtered through a D4 frit, and ligroin is added. On removal of the solvents using a rotary evaporator, the red product bis-N,N'-(2-methylene-15-crown-5)-perylene-3,4:9,10-bis(dicarboximide) precipitates out and, after thorough washing with diethyl ether and pentane, is dried for 24 hours at 100°C under an oil-pump vacuum. Yield 0.20 g (26 %), m.p. > 350°C. - R_{F} (silica gel/CHCl₃/triethylamine (10 + 1)) = 0.42 - IR (KBr): ν = 2910 cm⁻¹ (m sh), 2869 (m), 1694 (s), 1658 (s), 1618 (w), 1596 (s), 1579 (m), 1511 (w), 1506 (w), 1467 (w), 1439 (m), 1403 (m), 1358 (s br.), 1349 (s br.), 1301 (w br.), 1249 (m), 1178 (m), 1119 (s br.), 1036 (w), 985 (w), 946 (w), 865 (w), 811 (m), 795 (w), 747 (m), 640 (w). - UV (CHCl₃): λₘₐₓ(ε) = 526 nm (85 200), 489 (51 000), 458 (18 200). - Fluorescence (CHCl₃): λₘₐₓ(ε) = 534 nm, 574. - Solid-state fluorescence: λₘₐₓ(ε) = 625 nm. - C₄₆H₅₀N₂O₁₄ (854.9): calc. C 64.63, H 5.90, N 3.28; found C 64.54, H 5.91, N 3.33.

### Example 17: Bis-N,N'-(2-methylene-18-crown-6)-perylene-3,4:9,10-bis(dicarboximide)

0.27 g (0.69 mmol) of perylenetetracarboxylic acid bisanhydride, 0.44 g (1.50 mmol) of 2-aminomethyl-18-crown-6 and 5 g of imidazole are stirred at an oil bath temperature of 140°C. The reaction is terminated after 1.5 hours by the careful dropwise addition of 200 ml of 2N hydrochloric acid. The mixture is stirred for a further one hour at room temperature and then extracted with a total of 600 ml of chloroform (until the aqueous phase has become decoloured), and the organic phase is filtered over aluminium oxide (10·4 cm column)/chloroform/ethanol (10 + 1). Yield 0.53 g (82 %) of crude product. The crude product is chromatographed twice on silica gel/chloroform/triethyiamine (10+1), the middle fractions proving to be uniform. The phases that are pure according to thin-layer chromatography are concentrated completely by evaporation and then taken up in a small amount of chloroform and filtered through a D4 frit, and ligroin is added. When the solvent mixture is distilled off, bis-N,N'-(2-methylene-18-crown-6)-perylene-3,4:9,10-bis(dicarboximide) precipitates out and, after thorough washing with diethyl ether and pentane, is dried for 24 hours at 100°C *in vacuo* (0.013 mbar). 0.1 g of bis-N,N'-(2-methylene-18-crown-6)-perylene-3,4:9,10-bis(dicarboximide) is chromatographed again on silica gel/chloroform/triethylamine (10 + 1) (analytically pure). Yield 0.36 g (55 %), m.p. 290-291 °C. - R_{F} (silica gel/CHCl₃/triethylamine (10+1)) = 0.30. IR (KBr): ν = 2911 cm⁻¹ (m), 2865 (m), 1693 (s), 1653 (s), 1594 (s), 1577 (m), 1506 (w), 1472 (w), 1457 (w), 1440 (m), 1403 (m), 1352 (m br.), 1290 (w), 1250 (m), 1178 (w), 1103 (s br.), 990 (w), 959 (w), 860 (w), 835 (w), 810 (m), 795 (w), 747 (m), 668 (m). - UV (CHCl₃): λₘₐₓ(ε) = 526 nm (82 700), 490 (50 000), 459 (19 000). - UV (ethanol/water (4 + 1)): λₘₐₓ(ε) = 525 nm, 490, 459. - Fluorescence (CHCl₃): λₘₐₓ(ε) = 533 nm, 575. - Fluorescence (ethanol/water (4 + 1)): λₘₐₓ(ε) = 540 nm, 576. C₅₀H₅₈N₂O₁₆ (943.0): calc. C 63.68, H 6.20, N 2.97; found C 63.46, H 6.12, N 3.04.

### Example 18: 4',5'-Diaminobenzo-15-crown-5

0.30 g (0.91 mmol) of 4'-amino-5'-nitrobenzo-15-crown-5 is suspended under argon in 20 ml of absolute ethanol, and 0.14 g (2.80 mmol) of hydrazine hydrate is added. The suspension is heated to 40°C, and a spatula tip of the catalyst Pd/C (10 % Pd) is added in portions, whereupon the immediate evolution of nitrogen can be observed. Further catalyst is added in portions until no further evolution of gas occurs (about 2 hours); the mixture is then heated under reflux for a further one hour and, after cooling, the catalyst is filtered off. Chloroform has proved to be a suitable solvent. The filtrate rapidly changes colour in the air via yellow to yellow-green. The solvent is then removed *in vacuo* and the residue is dried for one hour. The crude product 4',5'-diaminobenzo-15-crown-5 is used for the following reaction without further working up.

### Example 19: Condensation of 4',5'-diaminobenzo-15-crown-5 with N-(1-nonyl-decyl)-perylene-3,4:9,10-tetracarboxylic acid 3,4-anhydride 9,10-imide

0.40 g (0.61 mmol) of N-(1-nonyl-decyl)-perylene-3,4:9,10-tetracarboxylic acid 3,4-anhydride 9,10-imide and 0.27 g (0.91 mmol) of unpurified 4',5'-diaminobenzo-15-crown-5 are stirred in 10 ml of quinoline at 180°C for 2 hours. The reaction is terminated by the addition of 50 ml of ethanol, and the mixture is introduced into 200 ml of 2N hydrochloric acid and stirred overnight at room temperature. The resulting precipitate is separated off over a D4 frit, washed thoroughly with water/methanol and dried for one hour at 110°C. Yield 0.36 g (64 %). The crude product is chromatographed twice on silica gel using chloroform/ethanol (10 + 1) as eluant. Two red by-products are separated off in the first runnings, while the violet dye remains adsorbed in the upper third of the column. By changing the eluant mixture (chloroform/ethanol (4 + 1)), it is possible to obtain the product from the column in the form of a very broad band. The elution is accelerated by flash chromatography. Yield 0.23 g (41 %). For the elemental analysis, 0.1 g of the dye is chromatographed on aluminium oxide using chloroform as eluant. The dye solution is filtered through a D4 frit; methanol is added and then the solvent mixture is removed and the black-violet powder is dried for 24 hours at 90°C *in vacuo* (0.13 mbar). Yield 0.09 g (90 %, based on the purification step). Yield 0.23 g (41 %), m.p. > 350°C. - R_{F} (silica gel/-CHCl₃/ethanol (4+1)) = 0.68.- IR (KBr): ν = 2924 cm⁻¹(s), 2854 (m), 1693 (s), 1653 (s), 1616 (w), 1593 (s), 1576 (w), 1545 (w), 1506 (w), 1484 (m), 1469 (w), 1448 (m), 1425 (w), 1396 (m), 1375 (m), 1356 (m), 1337 (s), 1315 (m), 1290 (s), 1250 (m), 1216 (m), 1170 (w), 1129 (m br.), 1050 (w), 976 (w), 938 (w), 850 (m), 807 (s), 745 (m), 668 (m). UV (CHCl₃): λₘₐₓ(ε) = 573 nm (32 000), 533 sh (27 000), 356 (13 700). C₅₇H₆₅N₃O₈ (920.2): calc. C 74.40, H 7.12, N 4.57; found C 74.75, H 7.10, N 4.54.

## Claims

1. A perylene of formula I 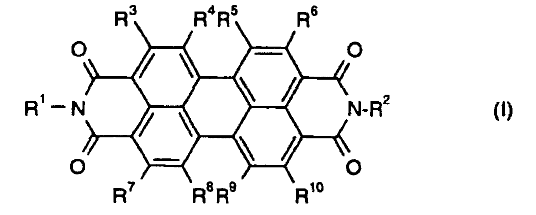 wherein
R¹ is 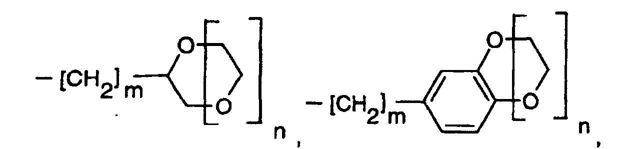 unsubstituted quinolyl, anthrolinyl or phenanthrolinyl or -OH-substituted quinolyl, -OH-substituted anthrolinyl or -OH-substituted phenanthrolinyl, wherein m is an integer from 0 to 4 and n is an integer from 1 to 9,
and the radicals R³ to R¹⁰ are hydrogen or a radical selected from the group consisting of an unsubstituted phenyl or phenyl which is substituted by halogen, cyano, -CN, unsubstituted C₁-C₁₈alkyl or C₁-C₁₈alkyl which is substituted by fluorine, cyano, -OCOR¹⁴, -OR¹², -OCOOR¹⁴, -CON(R¹²)(R¹³) or -OCONHR¹⁴; unsubstituted diphenyl or diphenyl which is substituted by halogen, cyano, -CN, unsubstituted C₁-C₁₈alkyl or C₁-C₁₈alkyl which is substituted by fluorine, cyano, -OCOR¹⁴, -OR¹², -OCOOR¹⁴, -CON(R¹²)(R¹³) or -OCONHR¹⁴; unsubstituted naphthyl or naphthyl which is substituted by halogen, cyano, -CN, unsubstituted C₁-C₁₈alkyl or C₁-C₁₈alkyl which is substituted by fluorine, cyano, -OCOR¹⁴, -OR¹², -OCOOR¹⁴, -CON(R¹²)(R¹³) or -OCONHR¹⁴; an unsubstituted heterocyclic aromatic radical having five to seven ring atoms or substituted heterocyclic aromatic radical having five to seven ring atoms which is substituted by halogen, cyano, -CN, unsubstituted C₁-C₁₈alkyl or C₁-C₁₈alkyl which is substituted by fluorine, cyano, -OCOR¹⁴, -OR¹², -OCOOR¹⁴, -CON(R¹²)(R¹³); halogen; unsubstituted C₁-C₁₈alkyl or C₁-C₁₈alkyl which is substituted by fluorine, cyano, -OCOR¹⁴, -OR¹², -OCOOR¹⁴, -CON(R¹²)(R¹³); -OR¹¹, -CN, -NR¹²R¹³, -COR¹⁴, -NR¹⁵COR¹⁴, -NR¹¹COOR¹⁴, -NR¹¹CONR¹²R¹³, -NHSO₂R¹⁴, -SO₂R¹⁴, -SOR¹⁴, -SO₂OR¹⁴, -CONR¹²R¹³, -SO₂NR¹²R¹³, -N=NR¹⁶, -OCOR¹⁴ and -OCONHR¹⁴, it being possible for two adjacent radicals together to form a carbocyclic or heterocyclic ring, wherein R¹⁴ is C₁-C₁₈alkyl, or C₆-C₁₀aryl, or benzyl that is unsubstituted or substituted by halogen, C₁-C₄alkyl or by C₁-C₄alkoxy, or is a 5- to 7-membered heterocyclic radical, R¹² and R¹³ are each independently of the other hydrogen, C₁- C₁₈alkyl, C₃- to C₂₄-cycloalkyl, C₆-C₁₀aryl or 5- to 7-membered heteroaryl, which are either unsubstituted or substituted by cyano or by hydroxy, or R¹² and/or R¹³ together with at least one of the other radicals R³ to R¹⁰ form a 5- or 6-membered heterocyclic ring,
R¹¹ is hydrogen, C₁-C₁₈alkyl, C₃- to C₂₄-cycloalkyl, C₆-C₁₀aryl or 5- to 7-membered heteroaryl,
R¹⁵ is hydrogen, C₁-C₁₈alkyl, C₃- to C₂₄-cycloalkyl, C₁-C₄alkylaryl, which are either unsubstituted or substituted by cyano, hydroxy or by C₁-C₄alkoxycarbonyl, C₆-C₁₀aryl, or a 5- to 7-membered heterocycle, which are unsubstituted or substituted by halogen, C₁-C₄alkyl or by C₁-C₄alkoxy,
R¹⁶ is the radical of a coupling component or is C₆-C₁₀aryl that is unsubstituted or substituted by halogen, C₁-C₄alkyl or by C₁-C₄alkoxy, and
R² is one of the radicals mentioned under R¹ or R³ to R¹⁰, with the proviso that R² is not R¹ if R¹ is unsubstituted or substituted quinolyl.

2. A perylene of the general formula II wherein the substituents are as defined in claim 1.

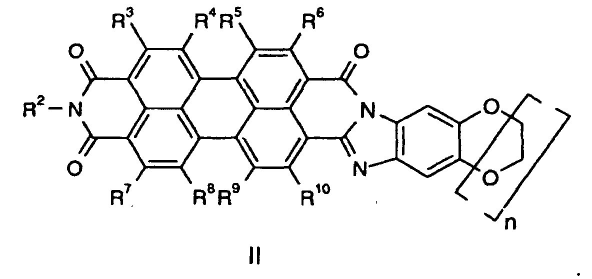

3. A process for the preparation of a perylene I according to claim 1 by reacting a perylene bisanhydride or a perylene anhydride imide with a primary amine at elevated temperature, in which process there is used as the primary amine a crown ether, substituted by an amino group, of the general formula III or IV 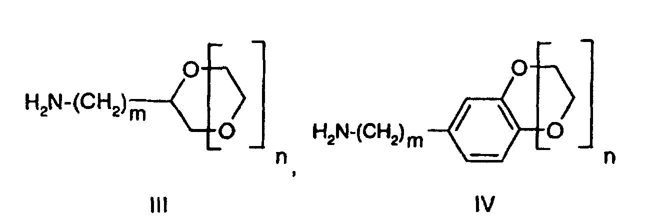 or a phenanthrolinyl radical of preferably formula V, an anthrolinyl radical of preferably formula VI or a quinolyl radical of preferably formula VII 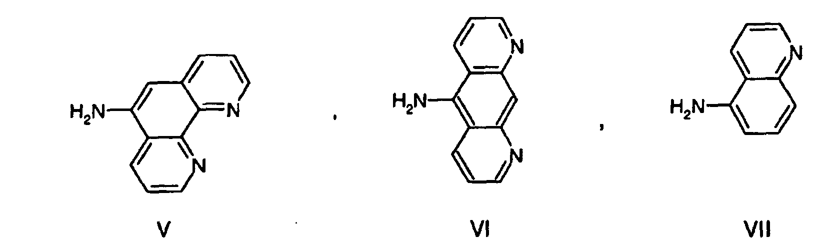 each of which is substituted by an amino group, it being possible for the radicals of formulae V to VII to contain further substituents, if desired.

4. A process for the preparation of a perylene II according to claim 2 by reacting a perylene anhydride imide with a diamine at elevated temperature, in which process there is used as the diamine the 4',5'-diaminobenzo-crown ether of formula VIII

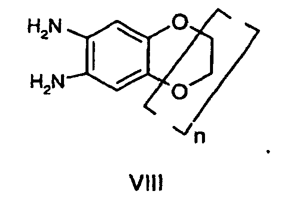

5. The use of a perylene I or II according to claims 1 or 2 as a detection reagent for heavy metal ions.

6. The use of a perylene I according to claim 1 wherein R¹ or R¹ and R² are the benzo-crown ether radical 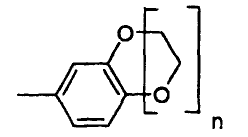 as a detection reagent for alkali metal and alkaline earth metal ions.

7. A metal ion complex of a perylene I or II according to claims 1 or 2.

8. A process for the preparation of a metal ion complex according to claim 7, which comprises bringing a metal salt, preferably a heavy metal salt, an alkali metal salt or an alkaline earth metal salt, if desired in the liquid phase, into contact with a perylene I or II.

9. The use of the perylenes I, II or their metal ion complexes according to claim 8 as colourants for the mass colouration of polymers, as vat dyes, as mordant dyes, in the preparation of paints, lacquers, especially automotive lacquers, coating compositions, paper dyes, printing inks, inks, especially for use in ink-jet printers, for drawing and writing purposes, and in electrophotography, for example for dry-copying systems (Xerox process) and laser printers, for security-marking purposes, as an additive to colourants, such as pigments and dyes, in which a particular shade of colour is to be achieved, for labelling objects for the purpose of mechanically recognising those objects by means of the fluorescence, for the frequency conversion of light, for the production of passive display elements for a wide variety of display, information and labelling purposes, as starting material for supraconducting organic materials, for solids fluorescent labelling, for decorative and artistic purposes, for tracer purposes, as fluorescent dyes in highly sensitive detection methods, as dyes or fluorescent dyes in optical light-collecting systems, in fluorescent solar collectors, in fluorescence-activated displays, in cold light sources for light-induced polymerisation in the preparation of plastics, for materials testing, in photoconductors, in photographic processes, in display, illumination or image-converting systems, as part of an integrated semiconductor circuit, which contain dyes as such or in conjunction with other semiconductors, in chemiluminescent systems, in luminescent immunoassays or other luminescent detection methods, as highlighter inks, in dye lasers, as an optical storage medium, as colour filters, as rheology improvers, as Q-switches; and as active substances for non-linear optics.

10. Mass coloured high molecular weight organic material selected from the group consisting of polymers polyvinyl chloride, cellulose acetate, polycarbonates, polyamides, polyurethanes, polyimides, polybenzimidazoles, melamine resins, silicones, polyesters, polyethers, polystyrene, polymethyl methacrylate, polyethylene, polypropylene, polyvinyl acetate, polyacrylonitrile, polybutadiene, polychlorobutadiene or polyisoprene, and the copolymers of the mentioned monomers, comprising a perylene I, II or a metal ion complex thereof according to claims 1, 2 or 7.

## Patentansprüche

1. Perylene der Formel I 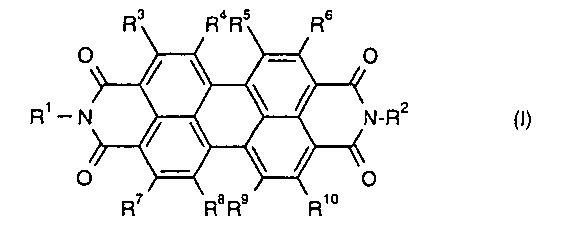 in der R' für 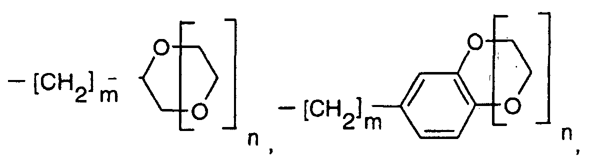 unsubstituiertes oder durch -OH substituiertes Chinolyl, unsubstituiertes oder durch -OH substituiertes Anthrolinyl, unsubstituiertes oder durch -OH substituiertes Phenanthrolinyl, wobei m eine ganze Zahl von 0 bis 4, und n eine ganze Zahl von 1 bis 9 bedeuten, und die Reste R³ bis R¹⁰ Wasserstoff, unsubstituiertes oder durch Halogen, Cyano, -CN, unsubstituiertem oder durch Fluor, Cyano, -OCOR¹⁴, -OR¹², OCOOR¹⁴, -CON(R¹²)(R¹³) oder -OCONHR¹⁴ substituiertem C₁-C₁₈-Alkyl substituiertes Phenyl, unsubstituiertes oder durch Halogen, Cyano, -CN, unsubstituiertem oder durch Fluor, cyano, -OCOR'⁴, -OR¹², OCOOR¹⁴, -CON(R¹²)(R¹³) oder -OCONHR¹⁴ substituiertem C₁-C₁₈-Alkyl substituiertes Diphenyl, unsubstituiertes oder durch Halogen, Cyano, -CN, unsubstituiertem oder durch Fluor, cyano, -OCOR¹⁴, -OR¹², OCOOR¹⁴, -CON(R¹²)(R¹³) oder -OCONHR¹⁴ substituiertem C₁-C₁₈-Alkyl substituiertes Naphthyl, unsubstituiertes oder durch Halogen, Cyano, -CN, unsubstituiertem oder durch Fluor, Cyano, -OCOR¹⁴, -OR¹², OCOOR¹⁴, -CON(R¹²)(R¹³) substituiertem C₁-C₁₈-Alkyl substituierter heterocyclischer aromatischer Rest mit 5 bis 7 Ringatomen, Halogen, unsubstituiertes oder durch durch Fluor, Cyano, -OCOR¹⁴, -OR¹², OCOOR¹⁴, -CON(R¹²)(R¹³) substituiertes C₁-C₁₈-Alkyl, OR¹¹, -CN, -NR¹²R¹³, -COR¹⁴, -NR¹⁵COR¹⁴, -NR¹¹COOR¹⁴, -NR¹¹CONR¹²R¹³, -NHSO₂R₁₄, -SO₂R¹⁴, -SOR¹⁴, -SO₂OR¹⁴, -CONR¹²R¹³, -N=NR¹⁶, -OCOR¹⁴ oder -OCONHR¹⁴, wobei je zwei benachbarte Reste zusammen einen carbocyclischen oder heterocyclischen Ring bilden können, wobei R¹² und R¹³ unabhängig voneinander für Wasserstoff, unsubstituiertes oder mit Cyano oder Hydroxi substituiertes
C₁-C₁₈-Alkyl, C₃- bis C₂₄-Cycloalkyl, C₆-C₁₀-Aryl oder 5- bis 7-gliedriges Heteroaryl stehen, oder worin R¹² und R¹³ miteinander oder R¹² und/oder R¹³ mit mindestens einem der anderen Reste R³ bis R¹⁰ einen 5- oder 6-gliedrigen heterocyclischen Ring bilden,
R¹¹ für Wasserstoff, C₁-C₁₈-Alkyl, C₃- bis C₂₄-Cycloalkyl, C₆-C₁₀-Aryl oder 5- bis 7-gliedriges Heteroaryl steht,
R¹⁵ für Wasserstoff, unsubstituiertes oder mit Cyano, Hydroxi oder C₁-C₄-Alkoxicarbonyl substituiertes C₁-C₁₈-Alkyl, C₃- bis C₂₄-Cycloalkyl, C₁-C₄-Alkylaryl, unsubstituiertes oder mit Halogen, C₁-C₄-Alkyl oder C₁-C₄-Alkoxi substituiertes C₆-C₁₀-Aryl, oder einen 5- bis 7-gliedrigen Heterocyclus steht,
R¹⁶ für den Rest einer Kupplungskomponente oder für unsubstituiertes oder mit Halogen, C₁-C₄-Alkyl oder C₁-C₄-Alkoxi substituiertes C₆-C₁₀-Aryl steht, und
R² für einen der unter R¹ oder R³ bis R¹⁰ genannten Reste steht, mit der Massgabe, dass R² ist nicht R¹, wenn R¹ unsubstituiertes oder substituiertes Chinolyl ist.

2. Perylene der allgemeinen Formel II 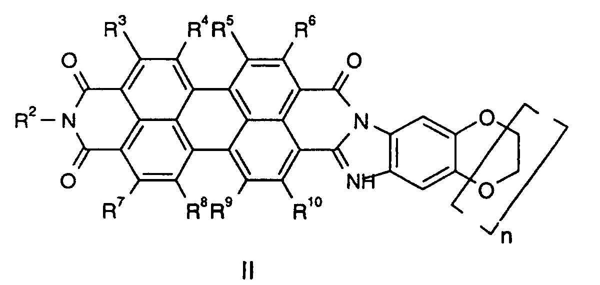 worin die Substituenten die in Anspruch 1 angegebene Bedeutung haben.

3. Verfahren zur Herstellung von Perylenen I gemäss Anspruch 1 durch Umsetzung eines Perylenbisanhydrids oder eines Perylenanhydridimids mit einem primären Amin bei erhöhter Temperatur, **dadurch gekennzeichnet, dass** man als primäres Amin einen mit einer Amino-Gruppe substituierten Kronenether der allgemeinen Formel III oder IV 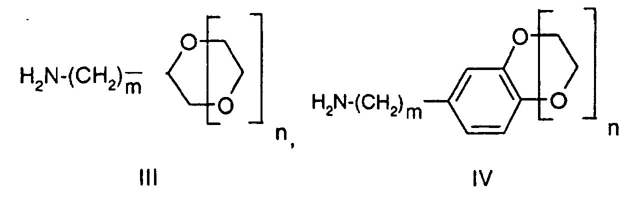 oder einen mit einer Amino-Gruppe substituierten Phenanthrolinyl-Rest, bevorzugt der Formel V, Anthrolinyl-Rest, bevorzugt der Formel VI oder Chinolyl-Rest, bevorzugt der Formel VII 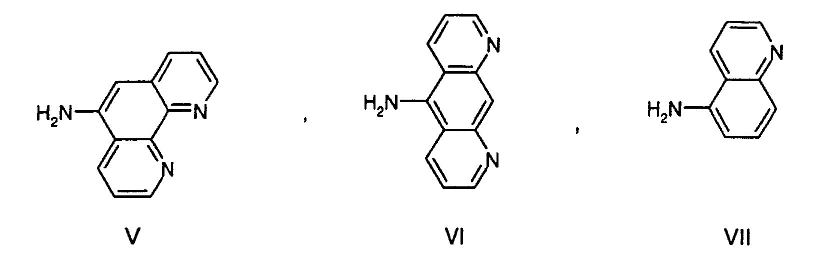 wobei die Reste der Formeln V bis VII gewünschtenfalls weitere Substituenten enthalten können, einsetzt.

4. Verfahren zur Herstellung der Perylene II gemäss Anspruch 2 durch Umsetzung eines Perylenanhydridimids mit einem Diamin bei erhöhter Temperatur, **dadurch gekennzeichnet, dass** man als Diamin den 4',5'-Diaminobenzo-Kronenether der Formel VIII 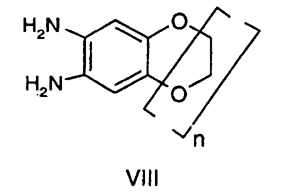 einsetzt.

5. Verwendung der Perylene I und 11 gemäss einem der Ansprüche 1 und 2 als Nachweisreagenz für Schwermetallionen.

6. Verwendung der Perylene I gemäss Anspruch 1, in denen R¹ oder R¹ und R² den Benzo-Kronenether-Rest 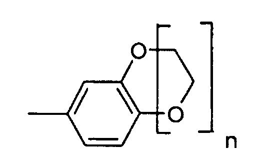 bedeuten, als Nachweisreagenz für Alkalimetall- und Erdalkalimetallionen.

7. Metallionen-Komplexe der Perylene I und II gemäss einem der Ansprüche 1 und 2.

8. Verfahren zur Herstellung der Metallionen-Komplexe gemäss Anspruch 7, **dadurch gekennzeichnet, dass** man ein Metallsalz, bevorzugt ein Schwermetall-, Alkalimetall- oder Erdalkalimetallsalz, gewünschtenfalls in flüssiger Phase mit den Perylenen I oder II in Kontakt bringt.

9. Verwendung der Perylene I, 11 und deren Metallionen-Komplexe gemäss Anspruch 8 als Farbmittel zur Masse-Färbung von Polymeren, als Küpenfarbstoffe, als Beizenfarbstoffe, zur Herstellung von Farben, Lacken, insbesondere Automobillacken, Anstrichstoffen, Papierfarben, Druckfarben, Tinten, insbesondere zum Einsatz in Tintenstrahldruckern, für Mal- und Schreib-Zwecke, sowie in der Elektrophotographie z.B. für Trockenkopiersysteme (Xerox-Verfahren) und Laserdrucker, für Sicherheitsmarkierungs-Zwecke, als Zusatz zu Farbmitteln wie Pigmenten und Farbstoffen, bei denen eine bestimmte Farbnuance erzielt werden soll, zum Markieren von Gegenständen zum maschinellen Erkennen dieser Gegenstände über die Fluoreszenz, zur Frequenzumsetzung von Licht, zur Herstellung von passiven Anzeigeelementen für vielerlei Anzeige-, Hinweis- und Markierungszwecke, als Ausgangsmaterial für supraleitende organische Materialien, zur Feststoff-Fluoreszenz-Markierung, für dekorative und künstlerische Zwecke, zu Tracer-Zwecken, als Fluoreszenzfarbstoffe in hochempfindlichen Nachweisverfahren, als Farbstoffe oder Fluoreszenzfarbstoffe in optischen Lichtsammelsystemen, in Fluoreszenz-Solarkollektoren, in Fluoreszenz-aktivierten Displays, in Kaltlichtquellen zur lichtinduzierten Polymerisation zur Darstellung von Kunststoffen, zur Materialprüfung, in Photoleitern, in fotografischen Verfahren, in Anzeige-, Beleuchtungs- oder Bildwandlersystemen, als Teil einer integrierten Halbleiterschaltung, die Farbstoffe als solche oder in Verbindung mit anderen Halbleitern enthalten, in Chemilumineszenzsystemen, in Lumineszenzimmunassays oder anderen Lumineszenznachweisverfahren, als Signalfarben, in Farbstoff-Lasern, als optisches Speichermedium, als Farbfilter, als Rheologieverbesserer, als Q-Switch-Schalter; sowie als aktive Substanzen für eine nichtlineare Optik.

10. In der Masse eingefärbtes hochmolekulares organisches Material ausgewählt aus der Gruppe bestehend aus Polymeren von Polyvinylchlorid, Celluloseacetat, Polycarbonaten, Polyamiden, Polyurethanen, Polyimiden, Polybenzimidazolen, Melaminharzen, Siliconen, Polyestern, Polyethern, Poiystyren, Polymethylmethacrylat, Polyethylen, Polypropylen, Polyvinylacetat, Polyacrylnitril, Polybutadien, Polychlorbutasien und Polyisopren und Copolymeren der entsprechenden Monomeren, enthaltend einen Perylene I, II oder ein Metallionen-Komplex davon gemäss den Ansprüchen 1, 2 oder 7.

## Revendications

1. Pérylène de formule I 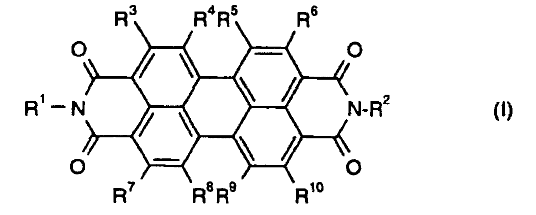 dans laquelle
R¹ représente : 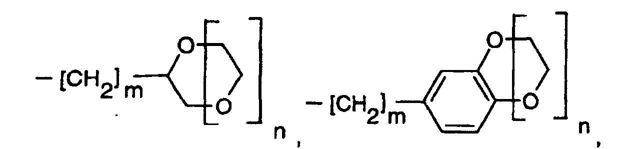 un groupe quinolyle, un groupe anthrolinyle ou phénanthrolinyle non substitué ou un groupe quinolyle substitué par un groupe -OH, un groupe anthrolinyle substitué par un groupe -OH ou phénanthrolinyle substitué par un groupe -OH, m étant un nombre entier de 0 à 4 et n étant un nombre entier de 1 à 9,
et les groupes R³ à R¹⁰ représentent un atome d'hydrogène ou un groupe choisi dans le groupe formé par un groupe phényle non substitué ou un groupe phényle qui est substitué par un atome d'halogène, un groupe cyano, -CN, alkyle en C₁ à C₁₈ non substitué ou alkyle eh C₁ à C₁₈ qui est substitué par un atome de fluor, un groupe cyano, -OCOR¹⁴, -OR¹², -OCOOR¹⁴, -CON(R¹²)(R¹³) ou -OCONHR¹⁴ ; un groupe diphényle non substitué ou un groupe diphényle qui est substitué par un atome d'halogène, un groupe cyano, -CN, alkyle en C₁ à C₁₈ non substitué ou alkyle en C₁ à C₁₈ qui est substitué par un atome de fluor, un groupe cyano, -OCOR¹⁴, -OR¹², -OCOOR¹⁴, -CON(R¹²)(R¹³) ou -OCONHR¹⁴ ; un groupe naphtyle non substitué ou un groupe naphtyle qui est substitué par un atome d'halogène, un groupe cyano, -CN, alkyle en C₁ à C₁₈ non substitué ou alkyle en C₁ à C₁₈ qui est substitué par un atome de fluor, un groupe cyano, -OCOR¹⁴, -OR¹², -OCOOR¹⁴, -CON(R¹²)(R¹³) ou -OCONHR¹⁴ ; un groupe aromatique hétérocyclique non substitué ayant cinq à sept atomes cycliques ou un groupe aromatique hétérocyclique substitué ayant cinq à sept atomes cycliques qui est substitué par un atome d'halogène, un groupe cyano, -CN, alkyle en C₁ à C₁₈ non substitué ou alkyle en C₁ à C₁₈ qui est substitué par un atome de fluor, un groupe cyano, -OCOR¹⁴, -OR¹², -OCOOR¹⁴, -CON(R¹²)(R13) ; un atome d'halogène ; un groupe alkyle en C₁ à C₁₈ non substitué ou alkyle en C₁ à C₁₈ qui est substitué par un atome de fluor, un groupe cyano, -OCOR¹⁴, -OR¹², -OCOOR¹⁴, -CON(R¹²)(R¹³) ; -OR¹¹, -CN, -NR¹²R¹³, -COR¹⁴, -NR¹⁵COR¹⁴, -NR¹¹COOR¹⁴, -NR¹¹CONR¹²R¹³, -NHSO₂R¹⁴, -SO₂R¹⁴, -SOR¹⁴, -SO₂OR¹⁴, -CONR¹²R¹³, -SO₂NR¹²R¹³, -N=NR¹⁶, -OCOR¹⁴ et -OCONHR¹⁴, deux groupes adjacents pouvant former conjointement un noyau carbocyclique ou hétérocyclique, où R¹⁴ représente un groupe alkyle en C₁ à C₁₈, aryle en C₆ à C₁₀ ou benzyle qui est non substitué ou substitué par un atome d'halogène, un groupe alkyle en C₁ à C₄ ou par un groupe alcoxy en C₁ à C₄, ou représente un groupe hétérocyclique à 5 ou 6 éléments,
R¹² et R¹³ représentent, indépendamment l'un de l'autre, un atome d'hydrogène, un groupe alkyle en C₁ à C₁₈, cycloalkyle en C₃ à C₂₄, aryle en C₆ à C₁₀ ou hétéroaryle de 5 à 7 éléments, qui sont non substitués ou substitués par un groupe cyano ou par un groupe hydroxyle, ou R¹² et/ou R¹³, conjointement avec au moins un des autres groupes R³ à R¹⁰, forment un noyau hétérocyclique de 5 ou 6 éléments,
R¹¹ représente un atome d'hydrogène, un groupe alkyle en C₁ à C₁₈, cycloalkyle en C₃ à C₂₄, aryle en C₆ à C₁₀ ou hétéroaryle de 5 à 7 éléments,
R¹⁵ représente un atome d'hydrogène, un groupe alkyle en C₁ à C₁₈, cycloalkyle en C₃ à C₂₄, (alkyle en C₁ à C₄)aryle, qui sont non substitués ou substitués par un groupe cyano, hydroxyle ou (alcoxy en C₁ à C₄)carbonyle, aryle en C₆ à C₁₀ ou un hétérocycle de 5 à 7 éléments, qui sont non substitués ou substitués par un atome d'halogène, un groupe alkyle en C₁ à C₄ ou alcoxy en C₁ à C₄,
R¹⁶ est le groupe d'un composant de couplage ou représente un groupe aryle en C₆ à C₁₀ qui est non substitué ou substitué par un atome d'halogène, un groupe alkyle en C₁ à C₄ ou par un groupe alcoxy en C₁ à C₄, et
R² est l'un des groupes mentionnés pour R¹ ou R³ à R¹⁰, à condition que R² soit différent de R¹ si R¹ représente un groupe quinolyle non substitué ou substitué.

2. Pérylène de formule générale II 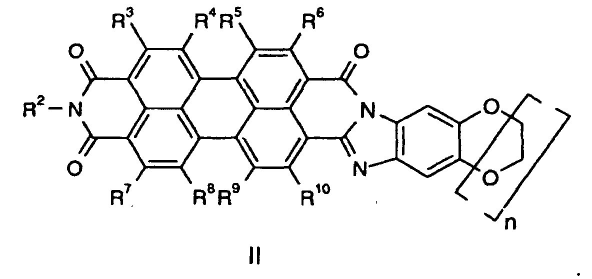 dans laquelle les substituants sont tels que définis dans la revendication 1.

3. Procédé de préparation d'un pérylène I selon la revendication 1, consistant à faire réagir un bisanhydride de pérylène ou un anhydride-imide de pérylène avec une amine primaire à température élevée, procédé dans lequel est utilisé, en tant qu'amine primaire, un éther couronne, substitué par un groupe amine, de formule générale III ou IV 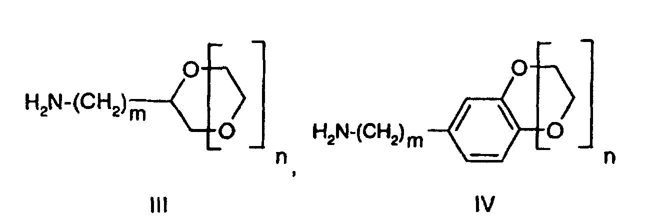 ou un groupe phénanthrolinyle, de préférence de formule V, un groupe anthrolinyle, de préférence de formule VI ou un groupe quinolyle, de préférence de formule VII 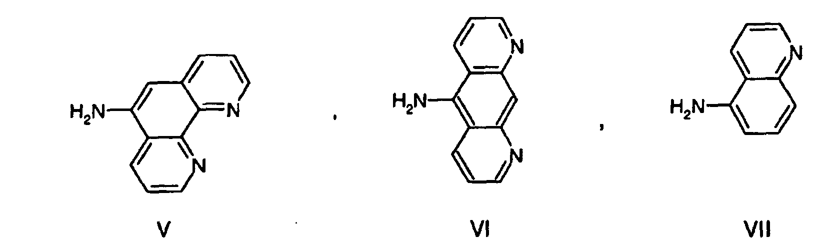 dont chacun est substitué par un groupe aminé, les groupes de formules V à VII pouvant contenir d'autres substituants, si souhaité.

4. Procédé de préparation d'un pérylène II selon la revendication 2, consistant à faire réagir un anhydride-imide de pérylène avec une diamine à température élevée, procédé dans lequel est utilisé, en tant que diamine, l'éther 4'-5'-diaminobenzo-couronne de formule VIII

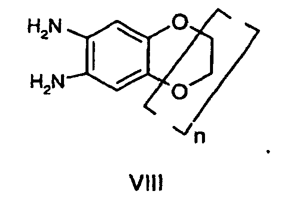

5. Utilisation d'un pérylène I ou II selon la revendication 1 ou 2 en tant qu'agent de détection d'ions de métal lourd.

6. Utilisation d'un pérylène I selon la revendication 1, dans lequel R¹ ou R¹ et R² sont le groupe éther benzo-couronne : 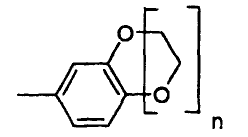 en tant qu'agent de détection d'ions de métal alcalin et de métal alcalino-terreux.

7. Complexe d'ions métalliques d'un pérylène I ou II selon la revendication 1 ou 2.

8. Procédé de préparation d'un complexe d'ions métalliques selon la revendication 7, comprenant l'étape consistant à mettre un sel métallique, de préférence un sel de métal lourd, un sel de métal alcalin ou un sel de métal alcalino-terreux, si souhaité en phase liquide, en contact avec un pérylène I ou II.

9. Utilisation des pérylènes I, II ou de leurs complexes d'ions métalliques selon la revendication 8, en tant que colorants pour la coloration en masse de polymères, en tant que colorants de cuve, en tant que colorants à mordant, dans la préparation des peintures, des laques, en particulier des laques automobiles, des compositions de revêtement, des colorants pour papier, des encres d'imprimerie, des encres, en particulier à utiliser dans les imprimantes à jet d'encre, à des fins de dessin et d'écriture, et en électrophotographie, par exemple pour les systèmes de tirage à sec (procédé Xerox) et les imprimantes laser, à des fins de marquage de sécurité, en tant qu'additif de colorants tels que les pigments et les matières colorantes, dans lesquels une nuance de couleur particulière doit être obtenue, pour marquer des objets dans le but de reconnaître mécaniquement ces objets par fluorescence, pour la conversion de fréquence d'une lumière, pour la production d'éléments d'affichage passifs pour une large diversité de buts d'affichage, d'information et de marquage, en tant que matière première pour matières organiques supraconductrices, pour le marquage par fluorescence de matières solides, à des fins décoratives et artistiques, à des fins de traçage, en tant que matières colorantes fluorescentes dans les procédés de détection hautement sensibles, en tant que matières colorantes ou matières colorantes fluorescentes dans les systèmes collecteurs de lumière optiques, dans les collecteurs solaires fluorescents, dans les affichages activés par fluorescence, dans les sources de lumière froide permettant d'induire par la lumière une polymérisation dans la préparation de matières plastiques, pour l'essai de matériaux, dans les photoconducteurs, dans les procédés photographiques, dans les systèmes d'affichage, d'illumination ou de conversion d'images, en tant que partie d'un circuit semi-conducteur intégré, qui contiennent des matières colorantes telles que ou conjointement avec d'autres semi-conducteurs, dans les systèmes chimioluminescents, dans les essais immunologiques par luminescence ou les autres procédés de détection par luminescence, en tant qu'encres de surligneurs, dans les lasers à colorants, en tant que milieu d'enregistrement optique, en tant que filtres couleur, en tant qu'agents améliorant la rhéologie, en tant que commutateur déclenché ; et en tant que substances actives pour systèmes optiques non linéaires.

10. Matière organique de masse moléculaire élevée colorée dans la masse, choisie dans le groupe formé par les polymères poly(chlorure de vinyle), l'acétate de cellulose, les polycarbonates, les polyamides, les polyuréthanes, les polyimides, les polybenzimidazoles, les résines mélamine, les silicones, les polyesters, les polyéthers, le polystyrène, le poly(méthacrylate de méthyle), le polyéthylène, le polypropylène, le poly(acétate de vinyle), le polyacrylonitrile, le polybutadiène, le polychlorobutadiène ou le polyisoprène, et les copolymères des monomères mentionnés, comprenant un pérylène I, II ou un de leurs complexes d'ions métalliques selon la revendication 1, 2 ou 7.
